# EUROPÄISCHE PATENTANMELDUNG

(11) **EP 0 832 898 A2**
(43) Veröffentlichungstag der Anmeldung: **01.04.1998**
(21) Anmeldenummer: 97114805.1
(22) Anmeldetag: 27.08.1997
(51) Int. Cl.: C07H 3/06, C07H 11/00, A61K 31/70

(54) **Substituierte Aminosäurederivate**

(30) Priorität: 09.09.1996 DE 19636538
(71) Anmelder: BAYER AG, 51368 Leverkusen (DE)
(72) Erfinder: Lockhoff, Oswald, Dr., 51373 Leverkusen (DE)

(57) **Zusammenfassung**

Die Erfindung betrifft mit Kohlenhydraten substituierte Aminosäuren der Formel 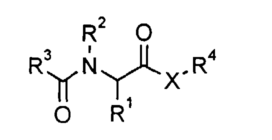 worin die Substituenten die in der Beschreibung angegebene Bedeutung haben, ihre Herstellung durch Mehrkomponentenreaktion und ihre Verwendung.

## Beschreibung

Die Erfindung betrifft mit Kohlenhydraten substituierte Aminosäuren, ihre Herstellung durch Mehrkomponentenreaktion und ihre Verwendung.

Die kombinatorische Chemie ist eine Technik, durch die mit hoher Effektivität in wenigen Syntheseschriften und innerhalb kurzer Zeit eine große Anzahl von unterschiedlichen Molekülen hergestellt werden kann, um sie dann beispielsweise einer pharmakologischen Prüfung zuzuführen. Als ein Hauptmerkmal der kombinatorischen Chemie ist dabei die Synthese der Verbindungen so angelegt, daß eine Reihe von Analogen unter ähnlichen Reaktionsbedingungen hergestellt werden kann. Dies ermöglicht den Einsatz von semi-automatisierten Prozessen. Das Prinzip der kombinatorischen Synthese einschließlich aktueller Anwendungen ist beispielsweise von Terrett et al. (*Tetrahedron*, **1995**, *51*, 8135-8173) zusammengefaßt worden.

Eine Reihe von kombinatorischen Bibliotheken ist in der jüngeren Vergangenheit aufgebaut worden. Der größte Teil der Bibliotheken basiert auf Oligonucleotiden (J. R. Wyatt et al., *Proc. Natl. Acad Sci*., **1994**, *91*, 1356-1360 und darin zitierte Beispiele) oder Peptiden (R. A. Houghten et al., *Nature*, **1991**, *354*, 84-86 oder R. A. Owens et al., *Biochem. Biophys. Res. Commun*. **1991**, *181*, 402-408). In jüngerer Zeit sind auch heterocyclische Bibliotheken erstellt worden, in denen zum Beispiel Benzodiazepine (B. A. Bunin und J. A. Ellman, *J*. *Am. Chem. Soc.*, **1992**, *114*, 10997-10998) oder Diketopiperazine (D. W. Gordon und J. Steele, *Biomed. Chem. Lett*., **1995**, *5*, 47-50) mit unterschiedlichen Substituenten versehen sind. Als prinzipiell zum Aufbau von chemischen Bibliotheken geeignet sind auch Kondensationsprodukte von Mehr-Komponenten-Reaktionen anzusehen (I. Ugi et al., *Endeavour*, **1994**, *18*, 115-123), die auf der Isonitrilchemie beruhen (G. Gokel et al., in *Isonitrile Chemistry*, I. Ugi, Hrsg., Academic Press, New York, 1971, S. 145-199). Die Vier-Komponenten-Reaktion ist auch mit Glycosylaminen als Amin-Komponenten durchgeführt worden (H. Kunz et al., *Synthesis*, **1991**, 1039-1042; S. Lehnhoff et al., *Angew. Chem*., **1995**, *107*, 1208-1211) und führt zu substituierten 2-Glycosylamino-acetamiden.

Auch Bibliotheken aus Kohlenhydratbausteinen sind in jüngerer Zeit beschrieben worden. Glycopeptid-Bibliotheken können in Festphasen-peptidsynthesen durch Verwendung von glycosylierten Aminosäurebausteinen aufgebaut werden (M. Meldal, *Curr. Opin. Struct. Biol.*, **1994**, *4*, 710-718). Oligosaccharidbibliotheken können durch Hydrazinolyse von natürlichen Glycokonjugaten erzeugt werden (Wing et al., *Glycoconjugate J.*, **1992**, *9*, 293-301). Chemische Verfahren zum Aufbau von Bibliotheken mit Kohlenhydratbausteinen, die über glycosidische Bindungen verknüpft sind, sind hingegen nur vereinzelt beschrieben worden. Ein solches Verfahren ist z.B. die "random glycosylation", die zu einem Gemisch von mehreren unterschiedlich verknüpften Di- und Trisacchariden führt (O. Hindsgaul et al., Angew. Chem. Int. Ed., **1996**, *34*, 2720-2722). Jedoch ist auch dieses Verfahren sorgsam zu optimieren und auf den jeweiligen Einzelfall abzustimmen. Dies ist zum Teil darauf zurückzuführen, daß für die stereoselektive Knüpfung einer spezifischen glycosidischen Bindung keine allgemein anwendbaren Verfahren existieren, wie dies bei der Synthese von Oligopeptiden oder -nucleotiden der Fall ist (Malik et al., *Chemiker-Ztg*., **1990**, *114*, 371-375). Deshalb gibt es auch keine allgemein anwendbaren Verfahren für Oligosaccharidsynthesen an der festen Phase, sondern lediglich spezielle Verfahren (L. Yan et al., *J. Am. Chem. Soc*., **1994**, *116*, 6953-6954). Polymer-unterstützte Synthesen von Oligosacchariden in Lösung nutzen die Vorteile der Glycosylierung in Flüssigphasen und die vereinfachten Aufarbeitungsverfahren von Festphasenreaktionen (J. J. Krepinsky et al., *Methods Enzymol*., **1994**, *242*, *Pt. A*, 280-293).

Die vorliegende Erfindung beschreibt die Synthese von entsprechend funktionalisierten Kohlenhydratbausteinen, die bei der Reaktion untereinander in einheitlicher Reaktion zu Di- und Oligosaccharid-Mimetika bzw. Glycokonjugat-Mimetika führen. Diese Reaktion ist geeignet, Bibliotheken von Kohlenhydrat-Mimetika zu generieren.

Die Erfindung betrifft Verbindungen der allgemeinen Formel (I) 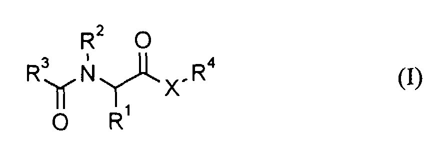 sowie pharmazeutisch akzeptable Salze davon,
in welcher
- R¹ und R²: gleich oder verschieden sein können und für einen gegebenenfalls substituierten, gesättigten oder einfach oder mehrfach ungesättigten Alkyl- oder Cycloalkylrest, einen gegebenenfalls substituierten Aryl- oder Aralkylrest, einen gegebenenfalls substituierten, gesättigten oder einfach oder mehrfach ungesättigten oder aromatischen Heterocyclus oder Heterocyclyl-alkylrest oder einen Rest -Y-R⁵ stehen,
- R³ und R⁴: gleich oder verschieden sein können und für Wasserstoff stehen oder die Bedeutung von R¹ aufweisen,
- X: für -O-, -NH-, -N(Niederalkyl)- oder -S- steht, wobei
- Y: für -(CH₂)ₙ-, -(CH₂)ₙ-O-, -(CH₂)ₙ-N(CO-R⁶)- oder -(CH₂)ₙ-CO-N(R⁷)- steht,
- R⁵: für einen substituierten Alkyl-, Tetrahydrofuranyl-, Tetrahydropyranyl-, Niederalkyl-tetrahydrofuranyl- oder Niederalkyl-tetrahydropyranyl-ring steht und
- R⁶ und R⁷: gleich oder verschieden sein können und für Wasserstoff oder Niederalkyl stehen und
- n: für eine Zahl von 0 bis 3 steht,
mit der Maßgabe, daß (i) die Verbindungen der Formel (I) mindestens einen Rest - Y-R⁵ aufweisen müssen, und (ii) in dem Falle, in dem R² für einen Glycosylrest steht, in den Verbindungen der Formel (I) mindestens ein zusätzlicher Rest -Y-R⁵ vorhanden sein muß.

Bevorzugt sind Verbindungen der Formel (I), in welcher gemäß obiger Beschreibung
- R⁵: für gegebenenfalls substituierte acyclische oder cyclische Kohlenhydratreste steht.

Die Verbindungen der Formel (I) enthalten ein oder mehrere Chiralitätszentren. Die Erfindung betrifft deshalb enantiomerenreine Verbindungen der Formel (I) als auch stereoisomere Formen davon.

Beispiele für Reste R¹, R², R³ oder R⁴ sind (i) Methyl, Ethyl, Propyl, iso-Propyl, Butyl, sek.-Butyl, tert.-Butyl, n-Hexyl, 5-Methyl-pentyl, 2-Ethyl-butyl, n-Octyl, 2-Ethyl-hexyl, n-Undecyl, n-Dodecyl, 1-Methyl-undecyl, 2,4-Dimethyl-decyl, 2,4,6-Trimethyl-nonyl oder n-Octadecyl; (ii) Vinyl, Allyl, But-2-enyl, But-3-enyl, Hex-2-enyl, Hex-3-enyl, Hex-4-enyl, Hex-5-enyl, Oct-2-enyl, Oct-4-enyl, Oct-6-enyl, Dodec-2-enyl, Dodec-2,4-dienyl, Hexadec-8-enyl, Octadec-8-enyl oder Octadadec-2,4,6,8-tetraenyl; (iii) Cyclopropyl, Cyclobutyl, Cyclopentyl, Cyclohexyl, Cycloheptyl, Cyclooctyl, Bicylo[3.1.1]hept-3-yl, Octahydropentalen-2-yl oder Octahydropentalen-5-yl; (iv) Cyclopent-1-enyl, Cyclopent-2-enyl, Cyclohex-1-enyl, Cyclohex-2-enyl oder Cyclohex-3-enyl; (v) Phenyl, Naphthalenyl oder Anthracenyl; (vi) Benzyl, Naphthalen-1-yl-methyl, Naphthalen-2-yl-methyl, Naphthalen-1-yl-ethyl, Naphthalen-2-yl-ethyl, Naphthalen-1-yl-butyl oder Anthracen-1-yl-methyl; (vii) Pyrrolyl, Pyrrolinyl, Pyrrolidinyl, Furanyl, Tetrahydrofuranyl, Thiophenyl, Indolyl, Benzofuranyl, Benzothiophenyl, Carbazolyl, Pyrazolyl, Imidazolyl, Oxazolyl, Thiazolyl, Isoxazolyl, Isothiazolyl, Triazolyl, Tetrazolyl oder Thiadiazolyl; (viii) Pyridinyl, Dihydropyridinyl, Tetrahydropyridinyl, Pyranyl, Dihydropyranyl, Tetrahydropyranyl, Chinolinyl, Isochinolinyl, Acridinyl, Chromanyl, Pyridazinyl, Pyrimidinyl, Piperazinyl, Pyranzinyl, Phenazinyl, Phenoxazinyl, Phenothiazinyl, Morpholinyl, Thiazinyl, Phenothiazinyl, Triazinyl, Purinyl oder Pteridinyl; (ix) Pyrrolyl-methyl, Pyrrolyl-ethyl, Pyrrolyl-propyl, Furanyl-methyl, Imidazolyl-ethyl, Thiazolyl-ethyl, Pyridinyl-methyl, Chinolinyl-methyl oder Phenothiazinyl-ethyl. Jeder dieser Reste kann für sich geeignet substituiert sein, beispielsweise durch Niederalkyl, Aza- oder Oxa- oder Thia-niederalkylen, Halogen, Trichlormethyl, Trifluormethyl, Niederalkoxy, Amino, Mono- oder Di-niederalkyl-substituiertes Amino, Amino-niederalkyl, Mono- oder Di-niederalkyl-aminoalkyl, Formyl, Nieder-alkanoyl, Niederalkanoyloxy, Carboxy, Niederalkoxycarbonyl, Nitrilo, Aminocarbonyl, Mono- oder Di-niederalkyl-amino-carbonyl, Nitro, Hydroxy, Hydroxymethyl, Hydroxyethyl, Phenyl, substituiertes Phenyl, Mercapto, Alkylmercapto, Cyano, Pyridyl, Sulfonyl, Sulfonyloxy, Phosphoryl oder Phosphoryloxy. Zwei Ringglieder der Substituenten können auch überbrückt sein, beispielsweise durch Methylen, Ethylen, Alkylen, freies oder ketalisiertes Carbonyl, freies oder verethertes oder verestertes Hydroxymethylen, -S-, -O-, -NH-CO-NH-, -O-CO-NH-, -NHSO₂- oder -CONHSO₂-.

Die Reste R⁵ stellen bevorzugt acyclische oder cyclische Kohlenhydrate oder Kohlenhydratderivate dar, in denen eine oder mehrere Hydroxygruppen durch Schutzgruppen substituiert sein können oder gegen Aminogruppen, Acylaminogruppen, Halogenatome, Wasserstoffatome oder Mercaptogruppen ausgetauscht sein können oder zu Ketogruppen oder Carboxylatgruppen oxidiert sein können. Die einzelnen Kohlenhydrate können auch durch weitere Glycosylreste substituiert sein, so daß einzelne oder mehrere Reste R⁵ aus Di- oder Oligosacchariden oder Derivaten davon bestehen können. Einzelne oder mehrere Hydroxygruppen können auch als Sulfat-, Phosphat- oder Nitratester vorliegen oder durch Carboxy-niederalkyl substituiert sein. Die cyclischen Kohlenhydrate R⁵ können sowohl der D- als auch der L-Reihe angehören. Die cyclischen Reste R⁵ können über das anomere Kohlenstoffatom oder über ein anderes Kohlenstoffatom des Kohlenstoffgerüstes gebunden sein.

Beispiele für substituierte acylische Alkylreste R⁵ sind 1,2,3-Trihydroxypropyl, 1,2,3,4-Tetrahydroxybutyl, 1,2,3,4,5-Pentahydroxypentyl oder 1,2,3,4,5,6-Hexahydroxyhexyl. Beispiele für Tetrahydrofuranylreste R⁵ und Niederalkyl-tetrahydrofuranylreste R⁵ sind Erythrofuranosyl, Threofuranosyl, Ribofuranosyl, Arabinofuranosyl, Xylofuranosyl, Allofuranosyl, Glucofuranosyl, Mannofuranosyl, Idofuranosyl, Galactofuranosyl, Talofuranosyl, Erythropentulfuranosyl, Fructofuranosyl, Altroheptulofuranosyl, Glyceromannooctulofuranosyl, Erythroglucononulofuranosyl, (Methyl-5-desoxy-ribofuranosid)-5-yl, (Methyl-6-desoxy-glucofuranosid)-6-yl, (Methyl-3-desoxy-glucofuranosid)-3-yl, (Methyl-3-desoxy-galactofuranosid)-3-yl, (Benzyl-3-desoxy-galactofuranosid)-3-yl. Beispiele für substituierte Tetrahydropyranylreste R⁵ und Niederalkyl-tetrahydropyranylreste R⁵ sind Ribopyranosyl, Arabinopyranosyl, Xylopyranosyl, Allopyranosyl, Glucopyransyl, Mannopyranosyl, Idopyranosyl, Galactopyranosyl, Talopyranosyl, Fucopyranosyl, Rhamnopyranosyl, Fructopyranosyl, Altro-heptulo-pyranosyl, Glycero-manno-octulo-pyranosyl oder Erythro-gluco-nonulo-pyranosyl, (Methyl-6-desoxy-glucopyranosid)-6-yl, (Methyl-3-desoxy-glucopyranosid)-3-yl oder (Methyl-4-desoxy-glucopyranosid)-4-yl.

Einzelne oder mehrere oder sämtliche Hydroxygruppen in den Resten R⁵ können ausgetauscht sein gegen andere Substituenten R⁵, beispielsweise gegen Wasserstoff, Niederalkyl, Halogen, Niederalkyloxy, Oxa-niederalkyloxy, Niederalkenyloxy, Cycloalkoxy, Tetrahydropyranyloxy, Arylalkyloxy, Heteroarylalkyloxy, Niederalkyloxy-arylalkyloxy, Alkylidendioxy, Benzylidendioxy, Niederalkanoyloxy oder Niederalkanoyloxy, welches durch Halogen substituiert ist, Arylcarbonyloxy oder Arylcarbonyloxy, welches durch Halogen oder Niederalkoxy substituiert ist, Niederalkylsulfonyloxy, Arylsulfonyloxy, Niederalkylarylsulfonyloxy, Oxo, Amino, Mono- oder Di-niederalkylamino, Formylamino, Niederalkanoylamino oder Niederalkanoylamino, welches durch Halogen substituiert ist, Niederalkynylamino, Arylalkylamino, Azido, Phthalimido, Nitryloxy, Mercapto, Niederalkylthio, Niederalkanoylthio, Carboxyniederalkyloxy, Niederalkoxycarbonylalkyloxy, Carboxy, Niederalkyloxycarbonyl, Niederalkenyloxycarbonyl, Benzyloxycarbonyl, Phosphonooxy, Dialkyloxy-phosphoryloxy, Dibenzyloxyphosphoryloxy, Sulfooxy, Alkyloxysulfonyloxy, Benzyloxysulfonyloxy oder Sulfamoyloxy, gegebenenfalls substituiertes Glycosyloxy oder gegebenenfalls substituiertes Di- oder Oligosaccharidyloxy.

Beispiele für acyclische substituierte Alkylreste R⁵ sind 1,2,3,4-Tetra-acetyloxy-butyl oder 1,2,3,4,5-Tetra-benzyloxy-pentyl. Beispiele für Kohlenhydratreste R⁵, in denen einzelne oder mehrere oder sämtliche Hydroxygruppen ausgetauscht bzw. substituiert worden sind, sind 2-Amino-2-desoxy-glucopyranosyl, 2-Amino-2-desoxy-galactopyranosyl, 2-Acetylamino-2-desoxy-glucpyranosyl, 2-Acetylamino-2-desoxy-galactopyranosyl, 2-Azido-2-desoxy-glucopyranosyl, 2-Desoxy-glucopyranosyl, 4-Desoxy-glucopyranosyl, 6-Chlor-6-desoxy-glucofuranosyl, 6-Desoxy-6-fluor-glucopyranosyl, 4-Chlor-4-desoxy-glucopyranosyl, Glucofuranos-3-ulosyl, 6-Thio-glucopyranosyl, 3-Thio-galactofuranosyl, 5-Acetylamino-3,5-didesoxy-glycero-galacto-2-nonulopyranosyl-onsäure, Methyl-(5-acetylamino-3,5-didesoxy-glycero-galacto-2-nonulopyranosyl)-onat, Glucuronopyranosyl, Galacturonofuranosyl, Cellobiosyl, Maltotriosyl, 3-O-(Carboxymethyl)-fucopyranosyl, 6-O-Phosphorylgalactopyranosyl, 2,3,4,6-Tetra-O-methyl-glucopyranosyl, 2,3,4,6-Tetra-O-acetylglucopyranosyl, 2,3,4,6-Tetra-O-benzyl-glucopyranosyl, 2,3,4,6-Tetra-O-benzyl-galactopyranosyl, 2,3,4,6-Tetra-O-benzyl-mannopyranosyl, 2,3,4-Tri-O-benzyl-rhamnopyranosyl, 2,3,4-Tri-O-benzyl-fucopyranosyl, 2,3-Di-O-allyl-4,6-di-O-benzyl-mannopyranosyl, 2,3-Di-O-acetyl-4,6-O-benzyliden-glucopyranosyl, 2,3,5,6-Di-O-isopropyliden-mannofuranosyl, Methyl-(5-acetylamino-4,7,8,9-tetra-O-acetyl-3,5-didesoxy-glycero-galacto-2-nonulopyranosyl)-onat oder 2,3,6,2',3',4',6'-hepta-O-acetyl-maltosyl. Beispiele für Kohlenhydratreste, die nicht über das anomere Zentrum gebunden sind, sind Glucopyranos-6-yl, Methyl-2,3,4-tri-O-acetyl-glucopyranosid-6-yl, Phenyl-2,3,6-tri-O-benzoyl-galactopyranosid-4-yl, 1,2:3,4-Di-O-isopropylidengalactopyranos-6-yl.

Die Verbindungen der Formel (I) können durch Vierkomponenten-Reaktionen (G. Gokel et al., in *Isonitrile Chemistry*, I. Ugi, Hrsg., Academic Press, New York, 1971, S. 145-199), die auf der Isonitrilchemie beruhen, hergestellt werden. Dabei ist bekannt, daß man in einer speziellen Ausführungsform dieser Reaktionen bei Verwendung von Carbonylverbindungen, Alkylaminen, Carbonsäuren und Isonitrilen zu substituierten 2-Acylamino-acetamiden gelangt.

Die erfindungsgemäßen Verbindungen der Formel (I) werden erhalten, indem man als Bausteine eine oder mehrere der oben genannten Komponenten als acyclische oder cyclische Kohlenhydrate oder Kohlenhydratderivate einsetzt. Die Bausteine sind prinzipiell bekannt.

Acylische Carbonylverbindungen, die sich von Kohlenhydraten ableiten, sind beispielsweise alle freien oder an den Hydroxygruppen geschützten Aldosen, beispielsweise 2,3,4,5,6-Penta-O-acetyl-glucose. Acyclische Carbonsäuren sind Glyconsäuren oder Glycuronsäuren bzw. ihre Derivate wie 2,3,4,5,6-Penta-O-acetylgluconsäure. Acyclische Aminoverbindungen, welche sich von Kohlenhydraten ableiten, sind Aminoalditole wie beipielsweise 1-Amino-1-desoxy-glucitol oder 1-Amino-1-desoxy-mannitol. Acyclische Isonitrile auf Basis von Kohlenhydraten sind z.B. 2,3,4,5,6-Penta-O-acetyl-1-desoxy-1-isonitrilo-glucitol oder 2,3,4,5,6-Penta-O-acetyl-1-desoxy-1-isonitrilo-galactitol.

Bausteine aus cyclischen Kohlenhydratderivaten, die über das anomere Kohlenstoffatom gebunden sind, sind (i) Glycosyl-aldehyde wie beispielsweise Glycosyl-carbaldehyde, Glycosyl-acetaldehyde,Glycosyl-propionaldehyde, Glycosyloxy-acetaldehyde, Glycosyloxy-propionaldehyde, N-(ω-Oxo-alkyl)-N-glycopyranosyl-carbonamide oder N-Glycosyl-ω-oxo-carbonamide (ii) Glycosylamine, (Glycosylalkyl)-amine, (Glycosyloxy-alkyl)-amine, N-(ω-Amino-alkyl)-N-glycopyranosylcarbonamide oder ω-Amino-N-glycosyl-carbonamide (iii) Glycosyl-carbonsäuren, Glycopyranosyl-essigsäuren, Glycopyranosyloxy-essigsäuren, Glycopyranosyloxypropionsäuren, N-Acyl-N-glycosyl-aminoessigsäuren, N-Acyl-N-glycosyl-aminopropionsäuren, N-Glycosyl-oxalamsäuren oder N-Glycosyl-malonaminsäuren und (iv) Glycosyl-isonitril, Glycosylalkyl-isonitril, Glycosyloxy-alkyl-isonitril oder N-Acyl-N-glycosyl-aminoalkyl-isonitril.

Bausteine aus cyclischen Kohlenhydratderivaten, die nicht über das anomere Kohlenstoffatom, sondern vielmehr am Ringsystem oder exocyclisch gebunden sind, sind O-aminoethylierte Kohlenhydrate wie beispielweise Methyl-3-O-(aminoethyl)-galactopyranosid oder O-carboxymethylierte Kohlenhydrate wie beispielsweise Methyl-3-O-(carboxymethyl)-galactopyranosid.

Geeignete Verfahren zur Herstellung der Kohlenhydratkomponenten für die Vierkomponentenreaktion sind prinzipiell bekannt. Sie bauen auf bekannten Kohlenhydrat-Vorstufen auf. Verfahren zur Umwandlungen der funktionellen Gruppen untereinander sind ebenfalls bekannt und können nach etablierten Methoden der organischen Chemie durchgeführt werden. Die genannten Reaktionen können mit einer Vielzahl von unterschiedlichen Schutzgruppen an den entsprechenden Kohlenhydratkomponenten durchgeführt werden. Im folgenden wird der Zugang zu ausgewählten Bausteinen am Beispiel von Aldohexosen erläutert.

Acyclische Aldehyde (AA), die sich von Kohlenhydraten ableiten, können beispielsweise aus den entsprechenden Thioacetalen hergestellt werden (J. Stanek, M. Cerny, J. Kocourek, J. Pacak, *The Monosaccharides,* S. 589, Academic Press, New York, 1963). Aldonsäuren (AB) können beispielsweise durch Oxidation aus den entsprechenden Aldosen hergestellt werden (J. Stanek, M. Cerny, J. Kocourek, J. Pacak, *The Monosaccharides*, S. 656, Academic Press, New York, 1963). 1-Amino-1-desoxy-alditole (AC) können beispielsweise durch reduktive Aminierung von Aldosen hergestellt werden (J. W. Long, G. N. Bollenback, *Methods Carbohydr. Chem.*, S. 79, Academic Press, New York, 1963). Peracylierte 1-Desoxy-1-isonitrilo-alditole (AD) können beispielsweise aus 2,3,4,5,6-Penta-O-acetyl-1-desoxy-1-formylamino-alditolen durch Dehydratisierung mit Phosgen erhalten werden.

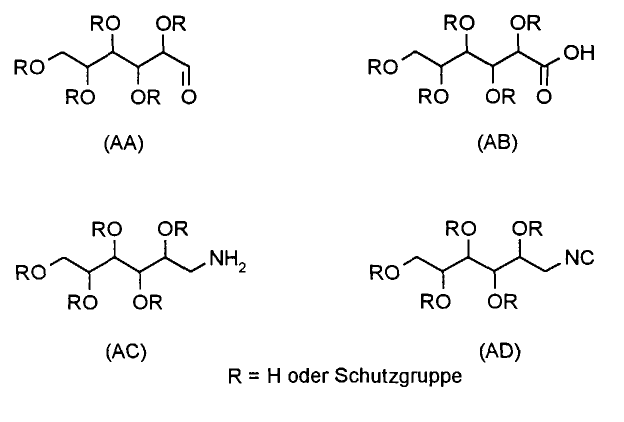

Glycosyloxy-acetaldehyde (BA) können beispielsweise durch Ozonolyse der entsprechenden bekannten Allylglycoside (Gigg, Gigg, *J. Chem. Soc. C* **1966**, 82) hergestellt werden (J. Lehmann, L. Ziser, *Carbohydr. Res.* **1992**, *225*, 83). Die Oxidation der Glycosyloxy-acetaldehyde (BA) liefert Glycosyloxy-essigsäuren (BB). Glycosyloxy-ethylamine (BC) können beispielsweise aus den entprechenden Bromethyl-glycosiden durch Umsetzung mit Azidionen zu Azidoethylglycosiden und deren Reduktion hergestellt werden. Glycosyloxy-ethylisonitrile (BD) können aus (BC) über die N-(Glycosyloxy-ethyl)-formamide und deren Dehydratisierung hergestellt werden.

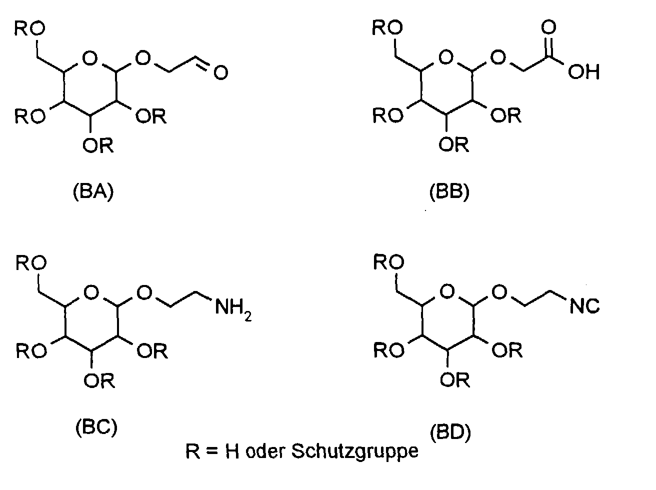

N-Acyl-N-glycosyl-aminoacetaldehyde (CA) sind durch Behandlung von N-Allyl-N-glycosyl-carbonamiden, welche nach bekannten Verfahren (O. Lockhoff, *Angew*. *Chem*. **1991**, *103,* 1639; W. Spevak, F. Dasgupta, C. J. Hobbs, J. O. Nagy, *J. Org. Chem.* **1996**, *61*, 3417) erhalten werden können, beispielsweise mit Ozon herzustellen. Deren Oxidation liefert nach bekannten Verfahren die N-Acyl-N-glycosylaminoessigsäuren (CB). Durch reduktive Aminierung in Gegenwart von Ammoniak sind aus (CA) die entsprechenden N-Acyl-N-glycosyl-ethylendiamine (CC) herstellbar. Aus (CC) wiederum lassen sich über die Stufe der N-Acyl-N-glycosylN 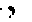 -formyl-ethylendiamine durch Dehydratisierung die entsprechenden N-Acyl-N-glycosyl-2-aminoethyl-isonitrile (CD) erhalten.

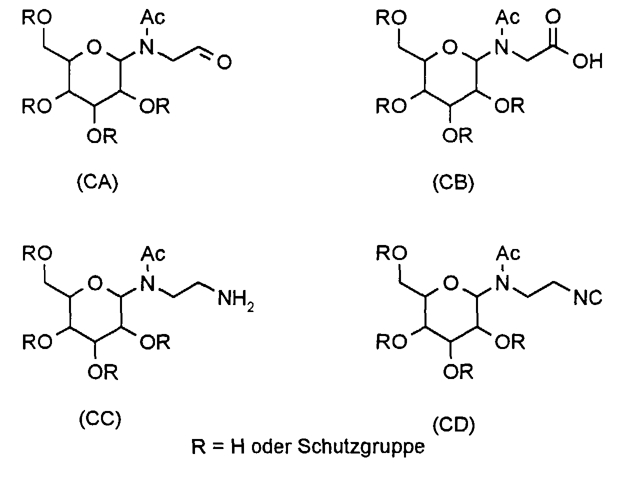

Aus N-Glycosyl-acrylamiden, welche aus Glycosylaminen und aktivierten Acrylsäurederivaten zugänglich sind (R. Roy, C. A. Laferriere, *J. Chem. Soc. Chem*. *Commun*. **1990**, 1709) sind durch oxidative Spaltung der Doppelbindung mit Ozon die N-Glycosyl-glyoxylsäureamide (DA) herzustellen. N-Glycosyl-oxalamsäuren können aus den entsprechenden Oxalamsäureestern durch Verseifung hergestellt werden. N-Glycosyl-2-aminoacetamide (DC) sind aus Glycosylaminen und N-geschützten Glycinderivaten unter den Bedingungen der Peptidsynthese und folgender N-Deblockierung zu erhalten. N-Glycosyl-2-isonitrilo-acetamide (DD) lassen sich aus (DC) über die entsprechende 2-Formylamino-acetamide und Dehydratisierung herstellen.

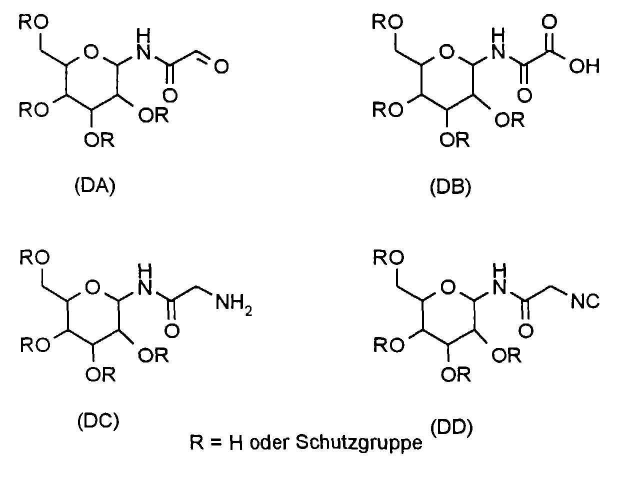

An den Hydroxygruppen substituierte Glycosylaldehyde (EA) sind bekannt (z.B.: Lasterra Sanchez et al., *Synlett*, **1994**, 705). Die Glycosylaldehyde (EA) können durch oxidative Prozesse, beispielsweise mit Kaliumpermanganat, in Glycosylcarbonsäuren (EB) übergeführt werden. Andererseits lassen sich Glycosylaldehyde (EA) mit Ammoniak oder Ammoniumsalzen unter reduktiven Bedingungen in Glycosylalkylamine (EC) überführen. Alternativ sind (EC) auch zu erhalten durch Reduktion der Glycosylaldehyde (EA) zu Glycosylcarbinolen, gefolgt von deren Umwandlung in Glycosylmethylsulfonate, Glycosylmethylazide und deren Reduktion zu Glycosylmethylaminen (EC). Letztere liefern nach Formylierung zu N-Glycosylmethyl-formamiden und Umsetzung mit dehydratisierenden Reagenzien wie beispielsweise Phosgen in Gegenwart von Basen wie DABCO die entsprechenden Glycosylmethyl-isonitrile (ED).

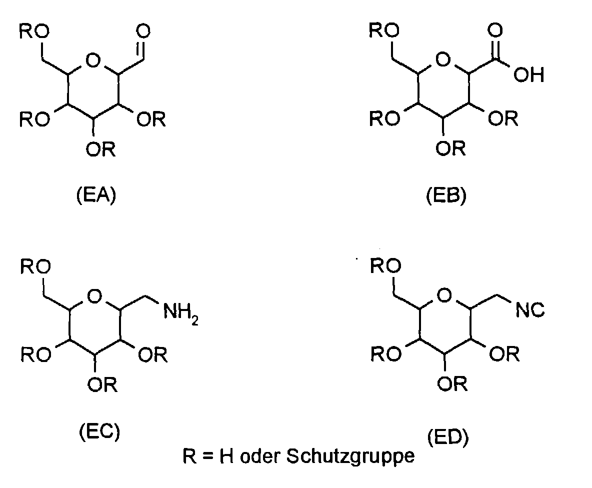

Ebenfalls bekannt sind Allyl-C-glycoside (D. M. Lewis, J. K. Cha, Y. Kishi, *J. Am. Chem. Soc*. **1982**, *104*, 4976). Durch Einwirkung von Ozon lassen sie sich in die Glycosyl-acetaldehyde (FA) überführen (W. W. McWorther, S. H. Kong, Y. Kishi, *Tetrahedron Lett*. **1983**, *24*, 2242). Entsprechend den oben genannten Verfahren lassen sie sich letztere in die Glycosyl-essigsäuren (FB) bzw. über die Stufe des 2-Glycosyl-ethanols in die Glycosyl-ethylamine (FC) und die Glycosyl-ethyl-isonitrile (FD) überführen.

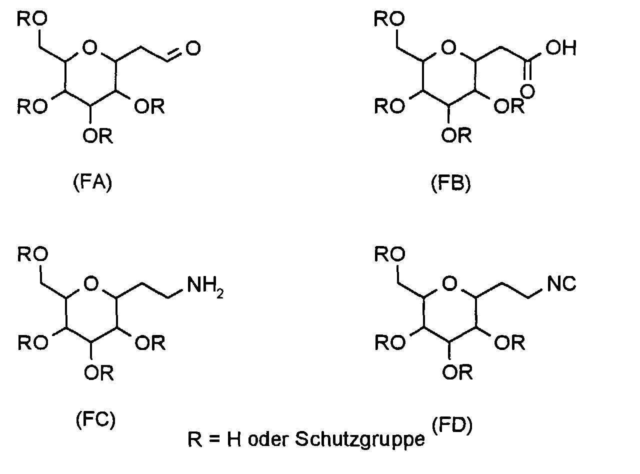

Es können auch solche Kohlenhydrat-Komponenten hergestellt werden, deren Aldehyd-, Carboxyl-, Amin- oder Isonitrilfunktion nicht an das anomere Zentrum des Zuckerrestes geknüpft ist. Diese Verbindungen sind bekannt oder sie können nach bekannten Verfahren hergestellt werden. So ist beispielsweise Methyl-2,3,4-tri-O-benzyl-D-glucopyranosid (Pravdic, Keglevic, *Tetrahedron* **1965**, *21*, 1897) ein geeignetes Ausgangsprodukt. Dessen Hydroxygruppe wurde zu dem Aldehyd (GA) oxidiert (Boren et al., *Acta Chem. Scand*. **1972**, *26*, 4143), der wiederum durch Oxidation zu der bekannten Carbonsäure (GB) führte (Pravdic, Keglevic, *Tetrahedron* **1965**, *21*, 1897). Aus der oben genannten Hydroxyverbindung ist auch das Amin (GC) erhältlich (Ueno et al., *Agric. Biol. Chem*. **1967**, *31*, 1346), das wiederum eine Vorstufe für das Isonitril (GD) ist.

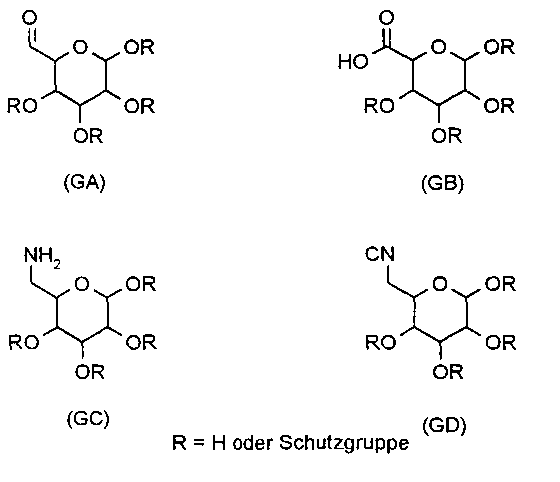

Als ein weiteres Beispiel für Kohlenhydrat-Bausteine mit Aldehyd-, Carboxylat-, Amino- und Isonitrilofunktionen, welche über eine kurze Alkylenkette an eine sekundäre Hydroxygruppe eines Zuckerderivats geknüpft sind, seien die folgenden Verbindungen genannt. Als Ausgangsverbindungen können Allylether von Kohlenhydraten verwendet werden, die bekannt sind (beispielsweise: Küster, Dyong, *Liebigs Ann. Chem*. **1975**, *2179* oder Varma, Schuerch, *J. Org. Chem*. **1981**, *46*, 799). Entsprechend den oben angeführten Verfahren sind daraus durch Ozonolyse die Aldehyde (HA) erhältlich, die oxidativ in die Carbonsäuren (HB) übergeführt werden können. Aus (HA) sind nach oben beschriebenen allgemeinen Verfahren die Amine (HC) erhältlich. Aus (HC) sind wiederum die Isonitrile (HD) zu erhalten.

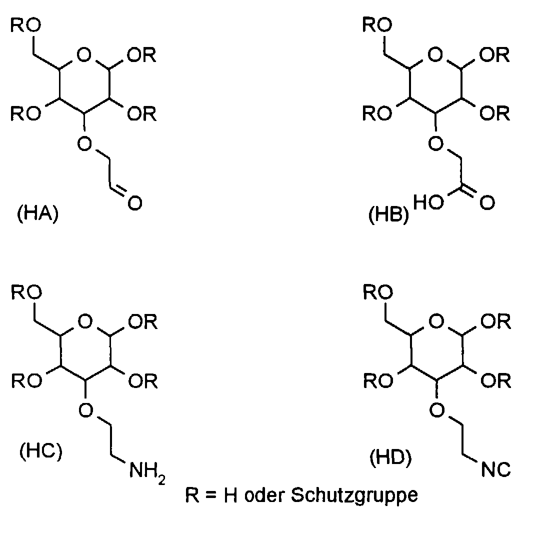

Mit Aminoethyl- oder Carboxymethyl-gruppen substituierte Kohlenhydrate lassen sich auch aus den entsprechenden O-Cyanomethylethern von Kohlenhydraten herstellen (C. Malet, O. Hindsgaul, *J. Org. Chem*. **1996**, *61*, 4649).

Die genannten Herstellungsweisen sind exemplarisch zu betrachten. Andere Möglichkeiten der Herstellung der angeführten Verbindungstypen sind dadurch nicht ausgeschlossen worden.

Desweiteren sind Glycosylamine in ungeschützter und in geschützter Form bekannt (H. Paulsen, K.-W. Pflughaupt in *The Carbohydrates* (W. Pigman, D. Horton, J. D. Wander, Hrsg.), 2. Aufl., Bd. 1B, Academic Press, New York ,**1980**). Ebenfalls bekannt sind Glycosyl-isonitrile (z.B. Boullanger et al. *Tetrahedron* **1979**, *35*, 163).

Es wurde nun überraschenderweise gefunden, daß die oben genannten Komponenten, deren Kohlenhydratkomponenten an ihren Hydroxyfunktionen wahlweise geschützt oder ungeschützt sind, geeignete Bausteine für Mehrkomponentenreaktionen sind. Dies wird im folgenden beispielhaft anhand der Komponenten (EA), (EB), (EC) und (ED) gezeigt, die beliebig mit anderen Aldehyden, Carbonsäuren, Aminen und Isonitrilen kombiniert werden können und zu Verbindungen der allgemeinen Formel (I) führen.

Bei der Reaktion des Glycosylaldehyds (EA) mit einem Amin R²-NH₂, einer Carbonsäure R³-COOH und einem Isonitril R⁴-NC wird in einheitlicher Reaktion eine Verbindung der Formel (II) erhalten.

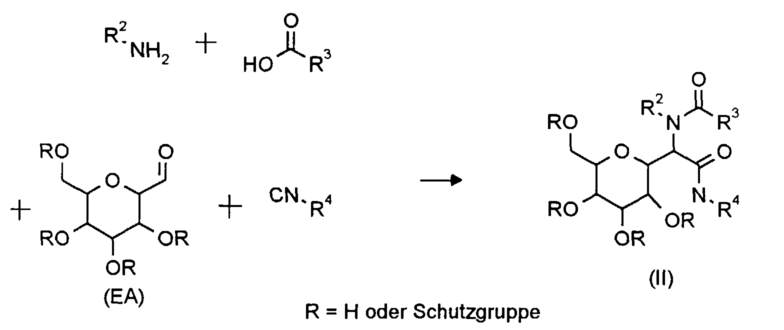

Entsprechend wird in einer Reaktion eines Aldehyds R¹-CHO, eines Amins R²-NH₂, der Glycosylcarbonsäure (EB) und eines Isonitrils R⁴-NC eine Verbindung der Formel (III) erhalten.

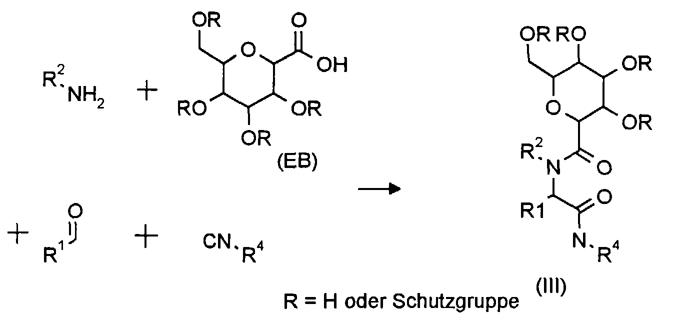

Analog wird bei der Reaktion eines Aldehyds R¹-CHO, eines Glycosylmethylamins (EC), einer Carbonsäure R³-COOH und eines Isonitrils R⁴-NC eine Verbindung der Formel (IV) erhalten.

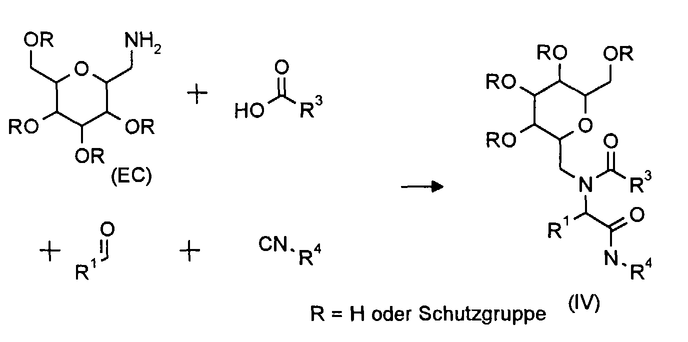

Entsprechend wird bei der Reaktion eines Aldehyds R¹-CHO, eines Amins R²-NH₂, einer Carbonsäure R³-COOH und eines Glycosylmethyl-isonitrils (ED) eine Verbindung der Formel (V) erhalten.

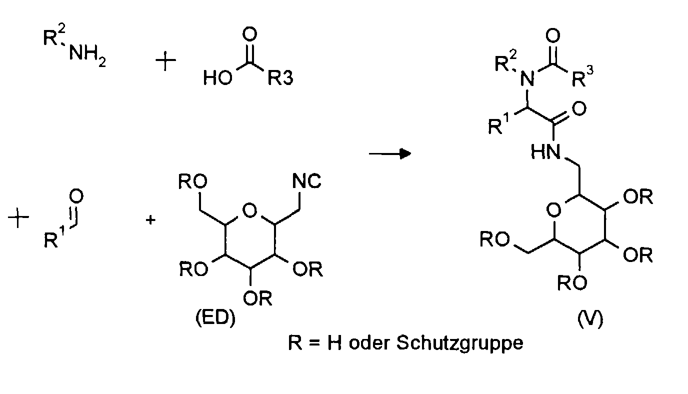

Darüber hinaus wurde gefunden, daß auch Mehrkomponenten-Reaktionen aus zwei oder mehr der o.g. Kohlenhydratderivate möglich sind. Beispielsweise wurde bei der Reaktion eines Glycosylaldehyds (EA), eines Glycosylmethylamin (EC), einer Carbonsäure R³-COOH und eines Isonitrils R⁴-NC eine Verbindung der Formel (VI) erhalten.

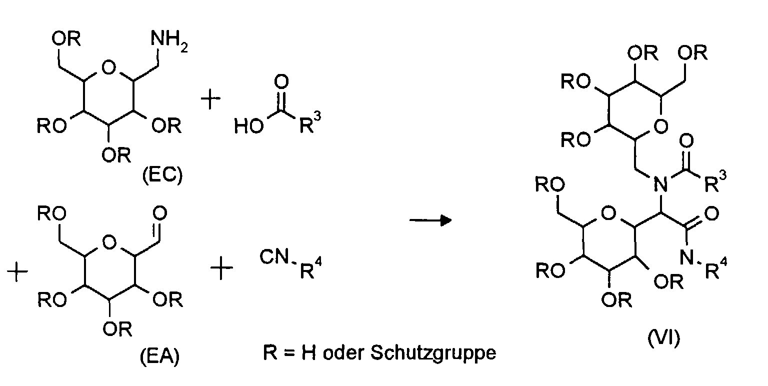

Entsprechend wird bei der Umsetzung eines Aldehyds (EA) mit einer Glycosylcarbonsäure (EB), einem Amin R²-NH₂ und einem Isonitril R⁴-NC eine Verbindung der Formel (VII) erhalten.

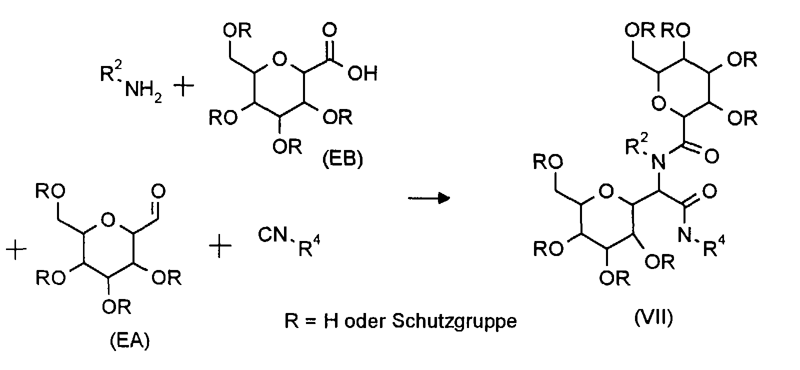

Entsprechend wird bei der Umsetzung eines Aldehyds (EA) mit einer Carbonsäure R³-COOH, einem Amin R²-NH₂ und einem Glycosylmethyl-Isonitril (ED) eine Verbindung der Formel (VIII) erhalten.

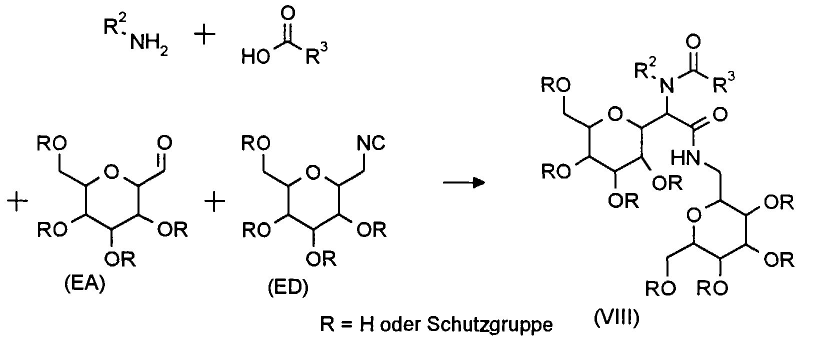

Entsprechend wird bei der Umsetzung eines Aldehyds R¹-CHO mit einem Glycosylmethyl-amin (EC), einer Glycosylcarbonsäure (EB) und einem Isonitril R⁴-NC eine Verbindung der Formel (IX) erhalten.

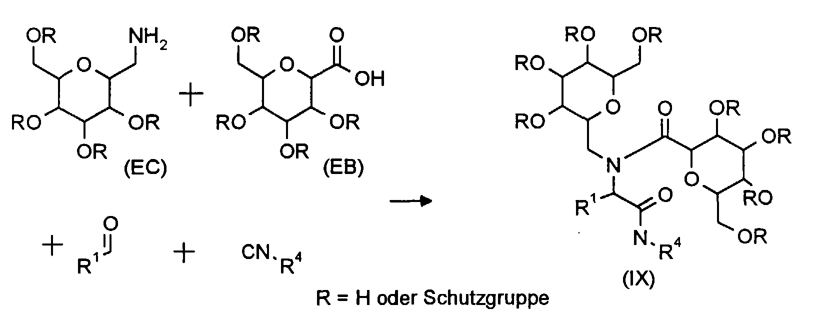

Entsprechend wird bei der Umsetzung eines Aldehyds R¹-CHO mit einem Glycosylmethyl-amin (EC), einer Carbonsäure R³-COOH und einem Glycosylmethylisonitril (ED) eine Verbindung der Formel (X) erhalten.

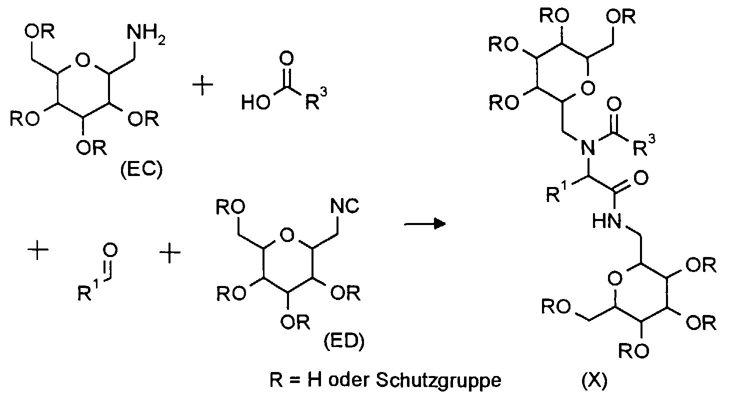

Entsprechend wird bei der Umsetzung eines Aldehyds R¹-CHO mit einem Amin R²-NH₂, einer Glycosylcarbonsäure (EB) und einem Glycosylmethyl-isonitril (ED) eine Verbindung der Formel (XI) erhalten.

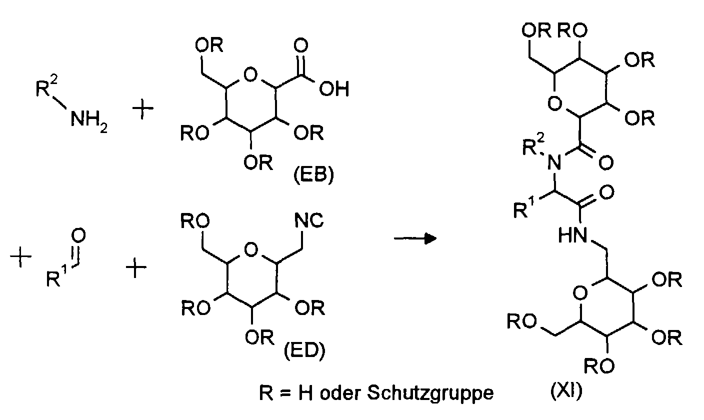

Es wurde darüber hinaus auch gefunden, daß sich durch Vierkomponenten-Reaktionen bei denen drei der vier Komponenten Kohlenhydratederivate sind, Kondensationsprodukte gebildet werden, welche drei Kohlenhydratreste enthalten. Analog zu den oben genannten Reaktionen wurden Verbindungen der Formel (XII), (XIII), (XIV) und (XV) erhalten.

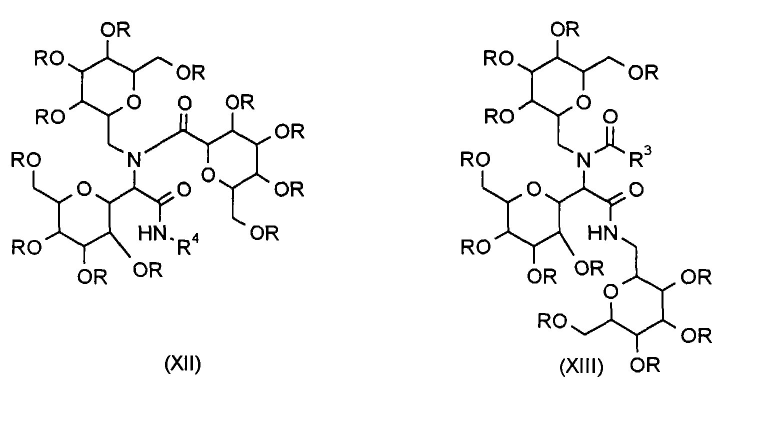

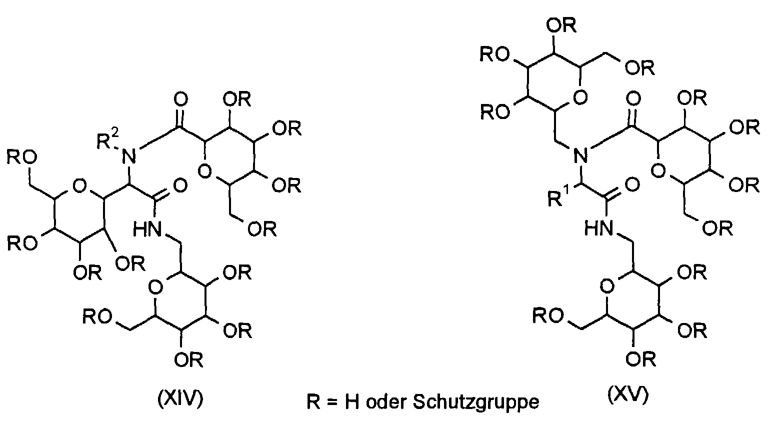

Darüber hinaus wurde gefunden, daß bei Einsatz von vier der oben genannten Kohlenhydratderivate, also dem Glycosylaldehyd (EA), der Glycosylcarbonsäure (EB), dem Glycosylmethyl-amin (EC) und dem Glycosylmethyl-isonitril (ED) ein Kondensationsprodukt mit vier Kohlenhydratresten der Formel (XVI) erhalten wurde.

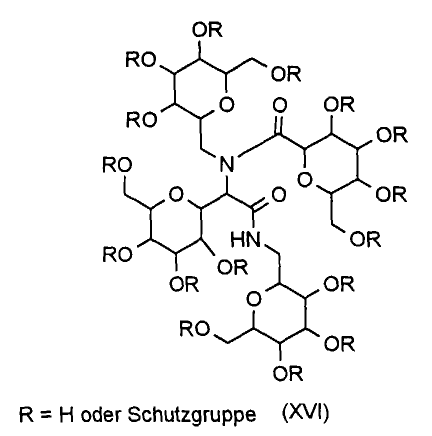

Darüber hinaus wurde gefunden, daß die erfindungsgemäßen Verbindungen der Formel (I) auch in einem mehrstufigen Verfahren erhalten werden können. Der Vorteil dieses Verfahrens besteht darin, daß spezielle Isonitrile von Kohlenhydraten oder mit anderen organischen Resten nicht zur Verfügung stehen müssen. Stattdessen können die Vierkomponentenreaktionen mit einem "universellen" Isonitril durchgeführt werden. Der mit diesem Isonitril eingeführte organische Rest, der in den Verbindungen der Formel (I) amidisch gebunden ist, wird dann im zweiten Schritt selektiv abgespalten, wodurch in Abhängigkeit der Reaktionsbedingungen eine freie Carbonsäurefunktion oder eine Carbonsäureesterfunktion erhalten wird. Die Carbonsäurefunktion ist wie bekannt funktionalisierbar und kann nach Aktivierung mit Aminen zu Amiden umgesetzt werden. Dieses Verfahren, das auf der Verwendung von 1-Isonitrilo-cyclohexen als "universellem" Isonitril beruht, ist bekannt (T. A. Keating, R. W. Armstrong, *J. Am. Chem. Soc.* **1996**, *118*, 2574). Es wird im folgenden beispielhaft beschrieben.

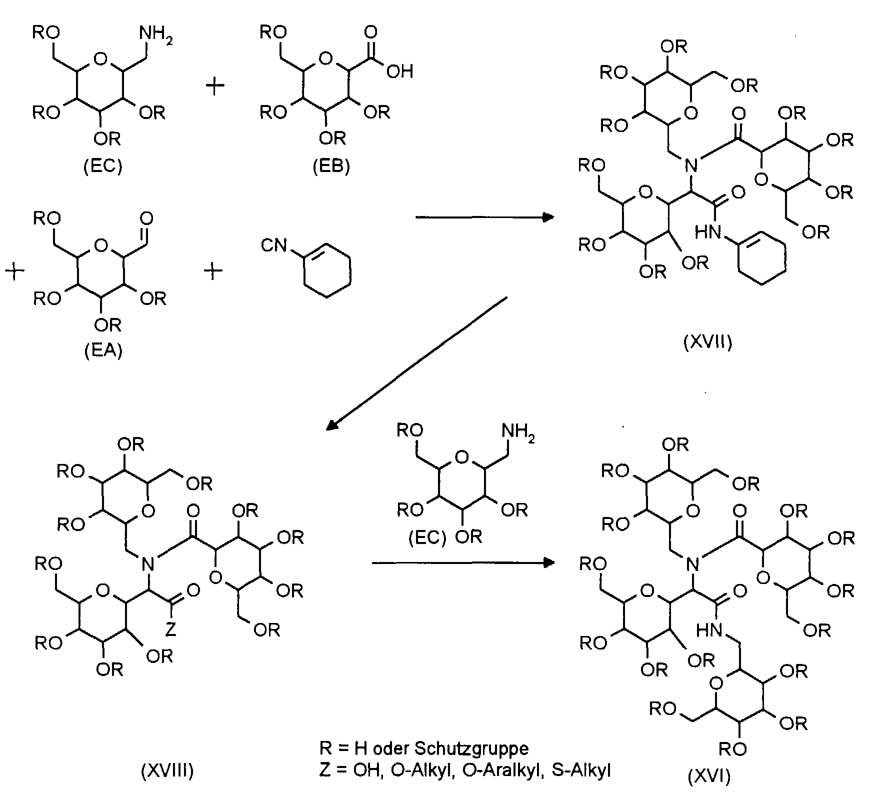

Die Umsetzung eines Glycosylaldehyds (EA) mit einem Glycosylmethylamin (EC), einer Glycosylcarbonsäure (EB) und 1-Isonitrilo-cyclohexen führt zu einem Kondensationsprodukt der Formel (XVII). Letzteres reagiert mit verdünnten Säuren zu Verbindungen der Formel (XVIII), wobei Z für OH steht. Mit Alkoholen oder Thiolen in Gegenwart von Säuren entstehen Verbindungen der Formel (XVIII), in der Z für O-Alkyl, O-Aralkyl oder S-Alkyl steht. Verbindungen der Formel (XVIII), in denen Z für OH steht, können mit Aminen, beispielsweise einem Glycosylmethylamin (EC) in Gegenwart wasserentziehender Mittel, wie sie aus der Peptidchemie bekannt sind, zu den schon weiter oben beschriebenen Acetamiden der Formel (XVI) umgesetzt werden. Die allgemeinen Bedingungen für die nach der Kondensation erfolgte Modifizierung der Reaktionsprodukte sind ebenfalls in der o.g. Literaturstelle beschrieben.

Die vier Komponenten der Reaktion werden vorteilhaft in etwa gleichen molaren Mengen oder mit geringen molaren Überschüssen, die im Bereich von 10-30 % liegen können, in die Reaktion zu den Produkten der Formel (I) eingesetzt. In diesem Fall geht man vorteilhaft so vor, daß man das Isonitril langsam zu der Mischung aus Aldehyd, Amin und Carbonsäure zudosiert. Anstelle des Amins und der Carbonylkomponente können auch deren Kondensationsprodukte, die Schiffschen Basen, mit gutem Erfolg mit Carbonsäuren und Isonitrilen zu den Produkten der Formel (I) umgesetzt werden.

Die vier Komponenten der Reaktion werden in einem geeigneten Lösungsmittel miteinander umgesetzt. Geeignete Lösungsmittel sind beispielsweise (i) Alkohole wie Methanol, Ethanol, n-Propanol, iso-Propanol, n-Butanol, iso-Butanol, tert.-Butanol, Hexanol, Octanol, Benzylalkohol, Ethylenglycol, Butylglycol oder Glycerin, (ii) Ether wie Diethylether, Methyl-tert.-butyl-ether, Tetrahydrofuran, Dioxan oder Diethylenglycoldimethylether, (iii) Ester wie Essigsäureethylester, (iv) Amide wie Dimethylformamid, N-Methyl-pyrrolidinon oder Hexamethylphosphorsäuretriamid, (v) halogenierte Kohlenwasserstoffe wie Dichlormethan, Chloroform oder 1,2-Dichlorethan, (vi) Kohlenwasserstoffe wie Hexan oder Toluol, (vii) Harnstoffe wie Tetramethylhamstoff oder Dimethyltetrahydropyrimidinon oder Nitromethan, Dimethylsulfoxid, Acetonitril oder Pyridin, die für sich alleine oder als Mischung untereinander oder zusammen mit Wasser verwendet werden können.

Die Reaktionstemperaturen können im Bereich von -78° bis +100° liegen. Sie sind auf die Nucleophilie des verwendeten Isonitrils abzustimmen. Dabei können die Reaktionszeiten im Bereich von wenigen Minuten bis hin zu einigen Tagen liegen.

Es kann von Vorteil sein, die Reaktion in Gegenwart von weiteren Zusätzen durchzuführen. Solche Zusätze können Metallsalze sein, beispielsweise Lithiumchlorid, Magnesiumbromid, Calciumchlorid, Eisenchlorid, Nickelchlorid oder Zinkchlorid.

Die bei den Reaktionen erhaltenen Produkte fallen als Rohmischungen an und werden in üblicher Weise aus den Reaktionsmischungen isoliert. Übliche Verfahren sind beispielsweise Kristallisation oder chromatographische Reinigungen.

In den Fällen, in denen geschützte Kohlenhydratkomponenten verwendet worden sind, können die Schutzgruppen selektiv von den Kohlenhydratgerüsten abgespalten werden. Methoden zur Abspaltung von Schutzgruppen sind bekannt (beispielsweise T. W. Greene: *Protective Groups in Organic Synthesis*, Wiley, New York, **1981**).

Durch Kombinationen ausgewählter Bausteine können Bibliotheken oligomerer Glycokonjugate aufgebaut werden. Diese Konjugate können Analoga von natürlich vorkommenden Oligosacchariden und Glycokonjugaten, sogenannte Kohlenhydrat-Mimetika, sein und somit mögliche Liganden von Kohlenhydrat-erkennenden Rezeptoren darstellen.

Es ist bekannt, daß die Oberfläche von Zellen mit Glycoproteinen und Glycolipiden ausgestattet ist, die in der interzellulären Kommunikation eine wichtige Bedeutung spielen. So sind die Glycokonjugate unter anderem Bindungsstellen für Bakterien, Toxine und Viren, sie sind essentiell bei der Befruchtung, sie sind antigene Determinanten und Liganden in der Zell-Zell-Adhäsion (A. Varki, *Glycobiology* **1993**, *3*, 97; G. W. Hart, *Curr. Opin Cell. Biol*. **1992**, *4*, 1017).

Inhibitoren der Kohlenhydrat-Protein-Wechselwirkung können als Arzneimittel von Bedeutung sein. Die erfindungsgemäßen Produkte sind potentiell zur Prävention oder zur Behandlung von Infektionskrankheiten, immunologischen Erkrankungen, Entzündungen, Krebs, Metastasierungen, Asthma und kardiovaskulären Erkrankungen von Bedeutung.

Oligosaccharide sind darüber hinaus für die Regulation der Bioverfügbarkeit von Wirkstoffen oder Proteinen von Bedeutung. Entsprechend sind die erfindungsgemäßen Produkte in konjugierter Form mit Wirkstoffen oder Proteinen für orale Aktivität, Toxizität und Spezifität, beispielsweise Gewebespezifität, potentiell von Bedeutung.

Die erfindungsgemäßen Verbindungen können auch als Modulatoren der Immunantwort, als Zell-Adhäsions-Moleküle und als Antigene von Bedeutung sein. Als Antigene können sie sowohl für immunologische Beeinflussungen des Immunsystems von Säugetieren, als Diagnostika und als Liganden von Immunoabsorbern von Bedeutung sein.

### Ausgangsverbindungen

*1. 2,6-Anhydro-3,4,5,7-tetra-O-benzyl-D-glycero-D-gulo-heptose:*
   Die Herstellung erfolgte entsprechend der Vorschrift von M. E. Lasterra Sanchez et al., *Synlett*, **1994**, 705 oder alternativ gemäß A. Dondoni und M. C. Scherrmann, *Tetrahedron Lett*. **1993**, *34*, 7319.
*2. 2,6-Anhydro-3,4,5,7-tetra-O-benzyl-D-glycero-D-gulo-heptonsäure:*
   2,6-Anhydro-3,4,5,7-tetra-O-benzyl-D-glycero-D-gulo-heptose (2.0 g, 3.69 mmol) wird in Aceton (72 ml) und Wasser (8 ml) gelöst und tropfenweise mit einer 0.14-molaren wässrigen Kaliumpermanganat-Lösung (33.6 ml, 4.71 mmol) versetzt. Nach Beendigung der Reaktion (DC-Kontrolle: Toluol-Aceton-Eisessig = 80:20:1) wird der Ansatz abgesaugt und das Filtrat eingeengt. Der Rückstand wird in Dichlormethan aufgenommen, mit Wasser gewaschen, getrocknet und eingeengt. Der erhaltene Sirup wird säulenchromatographisch an Kieselgel gereinigt (Laufmittel : Toluol-Aceton-Eisessig = 90:10:1). Ausbeute 1.13 g (55 %). C₃₅ H₃₆ O₇ [568.7]. Sirup, R_{f} = 0.22 (Toluol-Aceton-Eisessig = 80 : 20 : 1), [α]_{D} = +17.4° (c = 0.50, Dichlormethan), FAB-MS: *m/**z* 569 (*M* + H⁺), 567 (*M* - H⁺).
*3. 2,6-Anhydro-3,4,5,7-tretra-O-benzyl-D-glycero-D-gulo-heptitol:*
   2,6-Anhydro-3,4,5,7-tetra-O-benzyl-D-glycero-D-gulo-heptose (5.0 g, 9.05 mmol) wird in Tetrahydrofuran (100 ml) gelöst und bei 0°C tropfenweise mit einer 2M Lösung von Lithiumborhydrid in Tetrahydrofuran (4.6 ml, 9.05 mmol) versetzt. Nach 1 h bei 0°C auf 20°C erwärmt und 1 h nachgerührt. Der Ansatz wird auf Eiswasser gegeben, mit verd. Salzsäure angesäuert und mit Dichlormethan (200 ml) versetzt. Die organische Phase wird mit Wasser gewaschen, über Magnesiumsulfat getrocknet und eingeengt. Ausbeute Rohprodukt 5.0 g. Charakterisierung auf der Stufe des 1-O-Acetylderivats. C₃₅ H₃₈ O₆ [554.7]. R_{f} = 0.40 (Hexan-Essigester = 1 : 1), FAB-MS: *m/**z* 447 (*M* - BnOH + H⁺).
*4. 1-O-Acetyl-2,6-anhydro-3,4,5,7-tetra-O-benzy-D-glycero-D-gulo-heptitol:*
   2,6-Anhydro-3,4,5,7-tetra-O-benzyl-D-glycero-D-gulo-heptitol (10 mg, 0.02 mmol) wird in Pyridin (0.5 ml) gelöst und mit Acetanhydrid (0.25 ml) versetzt. Nach 16 h wird der Ansatz auf Eiswasser gegeben und mit Dichlormethan (3 ml) versetzt. Die organische Phase wird mit verd. Salzsäure, Wasser, gesättigter Natriumhydrogencarbonat-Lösung und Wasser gewaschen, über Magnesiumsulfat getrocknet und eingedampft. Der Rückstand wird säulenchromatographisch gereinigt (Laufmittel Hexan-Essigester 3:1). C₃₇ H₄₀ O₇ [596.7]. R_{f} = 0.39 (Hexan-Essigester = 2:1), CI-MS: *m*/*z* 597 (*M* + H⁺).
*5. 2,6-Anhydro-3,4,5,7-tetra-O-benzyl-1-O-tosyl-D-glycero-D-gulo-heptitol:*
   2,6-Anhydro-3,4,5,7-tetra-O-benzyl-D-glycero-D-gulo-heptitol (5.3 g, 9.6 mmol) werden in Pyridin (100 ml) gelöst und mit p-Toluolsulfonsäurechlorid (3.44 g, 18.0 mmol) versetzt. Nach 16 h wird auf Eiswasser gegossen, 15 min nachgerührt und mit Dichlormethan versetzt. Die organische Phase wird mit verd. Salzsäure, Wasser, gesättigter Natriumhydrogencarbonat-Lösung und Wasser gewaschen, getrocknet und eingeengt. Der Rückstand wird säulenchromatographisch gereinigt (Laufmittel Petrolether-Essigester 5:1). Ausbeute 6.1 g (90 %). C₄₂ H₄₄ O₈ S [708.9]. Schmp. 89 - 90°C, [α]_{D} = -3.8° (c = 0.39, Dichlormethan), R_{f} = 0.44 (Hexan-Essigester = 2 : 1), CI-MS: *m*/*z* 709 (*M* + H⁺).
*6. 2,6-Anhydro-1-azido-3,4,5,7-tetra-O-benzyl-1-desoxy-D-glycero-D-guloheptitol:*
   Die Lösung von 2,6-Anhydro-3,4,5,7-tetra-O-benzyl-1-O-tosyl-D-glycero-D-guloheptitol (6.0 g, 8.46 mmol) und Natriumazid (1.65 g, 25.4 mmol) in N,N-Dimethylformamid (100 ml) wird 2.5 h bei 80°C gerührt. Der Ansatz wird auf Raumtemperatur abgekühlt, mit tert.-Butylmethylether (200 ml) verdünnt, dreimal mit Wasser gewaschen, getrocknet und eingeengt. Der Rückstand wird säulenchromatographisch gereinigt (Laufmittel Hexan-Essigester 8:1). Ausbeute 3.6 g (73 %). C₃₅ H₃₇ N₃ O₅ [579.7]. Sirup, [α]_{D} = -21.5° (c = 0.46, Dichlormethan), R_{f} = 0.62 (Hexan-Essigester = 2:1). IR: 2120 cm⁻¹ (-N₃); CI-MS: *m*/*z* 552 (*M* - N₂ + H⁺).
*7. 1-Amino-2,6-anhydro-3,4,5,7-tetra-O-benzyl-1-desoxy-D-glycero-D-gulo-heptitol:*
   Die Lösung von 2,6-Anhydro-1-azido-3,4,5,7-tetra-O-benzyl-1-desoxy-D-glycero-D-gulo-heptitol (3.55 g, 6.42 mmol) und Natriumborhydrid (2.22 g, 58.77 mmol) in iso-Propylalkohol (35 ml) und Dioxan (7 ml) wird tropfenweise mit einer 4 %-igen (w/v) ethanolischen Nickelchlorid-Lösung (20 ml) versetzt. Es wird 2 h nachgerührt, dann abfiltriert, das Filtrat mit verd. Salzsäure angesäuert und mit Dichlormethan versetzt. Die Mischung wird mit verd. Natronlauge alkalisch gestellt. Die organische Phase wird abgetrennt, mit Wasser gewaschen, getrocknet und eingeengt. Ausbeute 3.18 g (90 %). C₃₅ H₃₉ N O₅ [553.7]. Sirup, [α]_{D} = +2.1° (c = 0.57, Dichlormethan), R_{f} = 0.13 (Dichlormethan-Methanol-konz. Ammoniak = 20 : 1.5 : 0.1), CI-MS: *m*/*z* 554 (*M* + H⁺).
*8. 2,6-Anhydro-3,4,5,7-tetra-O-benzyl-1-desoxy-1-formylamino-D-glycero-D-gulo-heptitol:*
   Die Lösung von 1-Amino-2,6-anhydro-3,4,5,7-tetra-O-benzyl-1-desoxy-D-glycero-D-gulo-heptitol (1.0 g, 1.81 mmol) in N,N-Dimethylformarnid (10 ml) wird mit Methylformiat (216 mg, 3.6 mmol) versetzt und 4 h auf 40°C erwärmt. Die Mischung wird im Hochvakuum eingeengt. Der Rückstand wird säulenchromatographisch gereinigt (Laufmittel Hexan-Essigester 3:1). Ausbeute 859 mg (81 %). C₃₆ H₃₉ N O₆ [581.7]. Sirup, [α]_{D} = +17.4° (c = 0.42, Dichlormethan), R_{f} = 0.84 (Dichlormethan-Methanol-konz. Ammoniak = 20:1.5:0.1). CI-MS: *m*/*z* 582 (*M* + H⁺).
*9. 2,6-Anhydra-3,4,5,7-tetra-O-benzyl-1-desoxy-1-isocyano-D-glycero-D-gulo-heptitol:*
   Die Lösung von 2,6-Anhydro-3,4,5,7-tetra-O-benzyl-1-desoxy-1-formylamino-D-glycero-D-gulo-heptitol (582 mg, 1.0 mmol) und DABCO (336.5 mg, 3.0 mmol) in Dichlormethan (10 ml) wird bei 0°C tropfenweise mit einer Lösung von Triphosgen (205 mg, 0.69 mmol) in Dichlormethan (10 ml) versetzt. Nach 30 min bei 0°C wird der Ansatz auf eine 0.5M Natriumcarbonat-Lösung (20 ml) gegossen und die organische Phase abgetrennt. Die organische Phase wird getrocknet und eingeengt. Der Rückstand wird säulenchromatographisch gereinigt (Laufmittel Petrolether-Essigester 7:1). Ausbeute 337 mg (60 %). C₃₆ H₃₇ N O₅ [563.7]. Sirup, [α]_{D} = -8.5° (c = 0.59, Dichlormethan), R_{f} = 0.86 (Hexan-Essigester = 1:1). IR: 2120 cm⁻¹ (-NC); CI-MS: *m*/*z* 564 (*M* + H⁺).
10. *2-(2,3,4,6-Tetra-O-benzyl-β-D-glucopyranosyl-oxy)-acetaldehyd:* In die Lösung von Allyl-2,3,4,6-tetra-O-benzyl-β-D-glucopyranosid (2.0 g, 3.44 mmol) in Dichlormethan (40 ml) wird bei -20°C Ozon eingeleitet. Nach beendeter Reaktion wird mit Dimethylsulfid (2 ml) versetzt und auf 20°C erwärmt. Die Lösung wird eingeengt und säulenchromatographisch gereinigt (Laufmittel Hexan-Essigester 3 : 1). Ausbeute 840 mg (42 %). C₃₆ H₃₈ O₇ [582.7]. Sirup, [α]_{D} = 5.9 ° (c = 0.44, Dichlormethan), R_{f} = 0.47 (Hexan-Essigester = 1 : 1). ESI-MS: *m*/*z* 605 (*M* + Na⁺).
*11. 2-(2,3,4,6-Tetra-O-benzyl-β-D-glucopyranosyl-oxy)-essigsäureethylester:*
   Die Lösung von 2,3,4,6-Tetra-O-benzyl-α-D-glucopyranosyl-trichloracetimidat (25.3 g, 37.0 mmol) und Ethylglycolat (3.5 ml, 37.0 mmol) in THF (100 ml) wird bei -30°C mit Bortrifluorid-Etherat (7.4 ml) versetzt. Nach 1 h wird auf eine gesättigte Natriumhydrogencarbonat-Lösung gegeben und mit Dichlormethan gewaschen. Die organische Phase wird mit Wasser gewaschen und eingeengt. Der Rückstand wird säulenchromatographisch gereinigt (Laufmittel Petrolether-Essigester 5 : 1). Ausbeute 18.9 g (81.7 %). C₃₈ H₄₂ O₈ [626.7]. Kristalle. Schmp. 46°C, [α]_{D} = 8.1 ° (c = 0.31, Dichlormethan), R_{f} = 0.23 (Hexan-Essigester = 3 : 1). ESI-MS: *m*/*z* 649 (*M* + Na⁺).
*12. 2-(2,3,4,6-Tetra-O-benzyl-β-D-glucopyranosyl-oxy)-essigsäure:* Die Lösung von 2-(2,3,4,6-Tetra-O-benzyl-β-D-glucopyranosyl-oxy)-essigsäureethylester (18.9 g, 30.2 mmol) in THF (200 ml) und Methanol (100 ml) wird mit 1N Natronlauge (20 ml) versetzt. Nach 1 h wird eingeengt, in Dichlormethan aufgenommen, mit 1N Salzsäure angesäuert, mit Wasser gewaschen, getrocknet und eingeengt. Kristallisation aus Dichlormethan-Petrolether. Ausbeute 7.1 g (39.3 %). C₃₆ H₃₈ O₈ [598.7]. Kristalle. Schmp. 120°C, [α]_{D} = 12.6 ° (c = 0.27, Dichlormethan), R_{f} = 0.26 (Dichlormethan-Methanol-Essigsäure = 10 : 1 : 0.1). ESI-MS: *m*/*z* 621 (*M* + Na⁺).
*13. N-(2,3,4,6-Tetra-O-benzyl-β-D-glucopyranosyl-oxy-ethyl)-formamid:* Die Lösung von 2,3,4,6-Tetra-O-benzyl-α-D-glucopyranosyl-trichloracetimidat (24.6 g, 36.0 mmol) und N-Hydroxyethyl-formamid (3.2 g, 36.0 mmol) in THF (100 ml) wird bei -30°C mit Bortrifluorid-Etherat (7.2 ml) versetzt. Nach 1 h wird auf eine gesättigte Natriumhydrogencarbonat-Lösung gegeben und mit Dichlormethan gewaschen. Die organische Phase wird mit Wasser gewaschen und eingeengt. Der Rückstand wird säulenchromatographisch gereinigt (Laufmittel Petrolether-Essigester 1 : 1). Der Rückstand wird aus Dichlormethan-Petrolether kristallisiert. Ausbeute 15.1 g (68.3 %). C₃₇ H₄₁ N O₇ [611.7]. Kristalle. Schmp. 126°C, [α]_{D} = 0.7 ° (c = 0.45, Dichlormethan), R_{f} = 0.14 (Hexan-Essigester = 1 : 1). ESI-MS: *m*/*z* 634 (*M* + Na⁺).
*14. 2-(2,3,4,6-Tetra-O-benzyl-β-D-glucopyranosyl-oxy)-ethylamin:* Die Lösung von N-(2,3,4,6-Tetra-O-benzyl-β-D-glucopyranosyl-oxy-ethyl)-formamid (1.2 g, 2.0 mmol) in THF (5 ml) und Methanol (5 ml) wird mit 1N Natronlauge (5 ml) versetzt und 1 h bei 20°C gerührt. Die Mischung wird eingeengt, in Dichlormethan aufgenommen, mit Wasser gewaschen, getrocknet und eingeengt. Der Rückstand wird säulenchromatographisch gereinigt (Laufmittel Dichlormethan-Methanol-Ammoniak_{conc} 30 : 1 : 0.1). Der Rückstand wird aus Dichlormethan-Petrolether kristallisiert. Ausbeute 0.77 g (66.0 %). C₃₆ H₄₁ N O₆ [583.7]. Kristalle. Schmp. 85°C, [α]_{D} = 7.2 ° (c = 0.61, Dichlormethan), R_{f} = 0.60 (Dichlormethan-Methanol-Ammoniak_{conc} 10 : 1.5 : 0.1). ESI-MS: *m*/*z* 584 (*M* + H⁺), 606 (*M* + Na⁺)
*15. 2-(2,3,4,6-Tetra-O-benzyl-β-D-glucopyranosyl-oxy)-ethylisonitril:* Die Lösung N-(2,3,4,6-Tetra-O-benzyl-β-D-glucopyranosyl-oxy-ethyl)-formamid (1.0 g, 1.64 mmol) und DABCO (0.55 g, 4.9 mmol) in Dichlormethan (20 ml) wird bei 0°C mit Triphosgen (0.33 g, 1.13 mmol) versetzt. Nach 30 min wird wird die Lösung auf gesättigte Natriumhydrogencarbonat-Lösung gegeben. Die organische Phase wird mit Wasser gewaschen, getrocknet und eingeengt. Der Rückstand wird säulenchromatographisch gereinigt (Laufmittel Petrolether-Essigester 5 : 1). Der Rückstand wird aus Dichlormethan-Hexan kristallisiert. Ausbeute 0.51 g (52.1 %). C₃₇ H₃₉ N O₆ [593.7]. Kristalle. Schmp. 79°C, [α]_{D} = 11.5 ° (c = 0.26, Dichlormethan), R_{f} = 0.31 (Hexan-Essigester 2 : 1). ESI-MS: *m*/*z* 594 (*M* + H⁺).

### Herstellungsbeispiele:

*A. Allgemeine Vorschrift zur Herstellung der erfindungsgemäßen Verbindungen der allgemeinen Formel (I) durch Vierkomponenten-Reaktion:*
   Die Mischung aus Aldehyd (0.5 mmol) und Amin (0.63 mmol) in Ethanol (1.5 ml) und Tetrahydrofuran (0.5 ml) wird 30 min bei Raumtemperatur gerührt, i. Vak. eingeengt, in Ethanol aufgenommen und eingeengt. Der Rückstand wird in Ethanol (2.5 ml) aufgenommen und mit Carbonsäure (0.63 mmol) und Isonitril (0.5 mmol) versetzt. Gegebenenfalls wird Zinkchlorid (0.65 mmol) zugesetzt. Die Mischung wird 12 bis 48 h gerührt (DC Kontrolle der Reaktion). Es wird 1N Salzsäure (0.25 ml) zugegeben, 30 min gerührt, auf Wasser gegeben und mit Dichlormethan (2.5 ml) versetzt. Die organische Phase wird 2mal mit wenig Wasser gewaschen, getrocknet und eingeengt. Der Rückstand wird säulenchromatographisch gereinigt.
*A-1. 2-[Acetyl-(4-methoxy-benzyl)-amino]-N-cyclohexyl-2-(2,3,4,6-tetra-O-benzyl-β-D-glucopyranosyl)-acetamid:*
   Aus 2,6-Anhydro-3,4,5,7-tetra-O-benzyl-D-glycero-D-gulo-heptose (277 mg, 0.5 mmol), 4-Methoxybenzylamin (86 mg, 0.63 mmol), Essigsäure (38 mg, 0.63 mmol) und Cyclohexylisonitril (54 mg, 0.5 mmol). Säulenchromatographische Reinigung (Laufmittel Hexan-Essigester = 3:1). Ausbeute Diastereomer A: 156 mg (37 %); Diastereomer B: 117 mg (28 %). *Diastereomer A*: C₅₂ H₆₀ N₂ O₈ [841.1]. Sirup, [α]_{D} = -9.2° (c = 0.92, Dichlormethan), R_{f} = 0.38 (Toluol-Ethanol = 10:1). CI-MS: *m*/*z* 841 (*M* + H⁺); ¹³C-NMR (100.58 Mhz, CDCl₃): 173.71, 173.49, 167.90, 167.56 (-CO-), 159.10, 158.63 (C-4 von p-Methoxybenzyl), 138.27, 138.13, 137.97, 137.72 (ipso-Ph), 130.81 - 125.85 (arom. CH und C-1 von p-Methoxybenzyl), 113.90 (C-3, C-5 von p-Methoxybenzyl), 87.40, 79.77, 79.28, 78.75, 78.44, 78.18, 77.24, 75.42, 75.16, 74.91, 74.64, 74.27, 73.52, 73.28, 69.86, 69.31, 68.96, 68.86 (C-1 - C-6 von Glycosyl und CH₂-Ph), 61.97 (C-2 von Acetamid), 55.30 (O-CH₃), 48.25 (C-1 von Cyclohexyl), 32.56, 32.34 (C-2, C-6 von Cyclohexyl), 25.45, 24.71 (C-3, C-4, C-5 von Cyclohexyl), 22.58 (CO-CH₃). *Diastereomer B*: C₅₂ H₆₀ N₂ O₈ [841.1]. Sirup, [α]_{D} = +25.3° (c = 0.45, Dichlormethan), R_{f} = 0.31 (Toluol-Ethanol = 10:1). CI-MS: *m*/*z* 841 (*M* + H⁺); ¹³C-NMR (100.58 Mhz, CDCl₃): 172.32, 166.75 (-CO-), 158.52 (C-4 von p-Methoxybenzyl), 138.30, 137.71 (ipso-Ph), 130.65 - 126.56 (arom. CH und C-1 von p-Methoxybenzyl), 113.99, 113.80 (C-3, C-5 von p-Methoxybenzyl), 86.93, 79.84, 79.38, 79.18, 78.57, 77.53, 77.24, 75.72, 75.61, 75.19, 75.03, 73.47, 68.43, 68.04 (C-1 - C-6 von Glycosyl und CH₂-Ph), 61.02, 60.63 (C-2 von Acetamid), 56.98, 55.27 (O-CH₃), 49.78, 48.34 (C-1 von Cyclohexyl), 32.78, 32.62, 31.92, 30.33 (C-2, C-6 von Cyclohexyl), 25.38, 24.73 (C-3, C-4, C-5 von Cyclohexyl), 22.69, 22.24 (CO-CH₃).
*A-2. 2-[Acelyl-(2,3,4,6-tetra-O-benzyl-β-D-glucopyranosyl-methyl)-amino]-N-cyclohexyl-2-phenyl-acetamid:*
   Aus Benzaldehyd (53 mg, 0.5 mmol), 1-Amino-2,6-anhydro-3,4,5,7-tetra-O-benzyl-1-desoxy-D-glycero-D-gulo-heptitol (349 mg, 0.63 mmol), Essigsäure (38 mg, 0.63 mmol) und Cyclohexylisonitril (54 mg, 0.5 mmol). Säulenchromatographische Reinigung (Laufmittel Petrolether-Essigester = 3:1). Ausbeute 260 mg (64 %). Es wurden zwei nicht trennbare Diasteromere erhalten, die jeweils als Rotamere vorliegen. C₅₁ H₅₈ N₂ O₇ [811.0]. Sirup, [α]_{D} = -16.9° (c = 0.36, Dichlormethan), R_{f} = 0.37 (Hexan-Essigester = 1:1). CI-MS: *m*/*z* 811 (*M* + H⁺); ¹³C-NMR (100.58 MHz, CDCl₃): 172.69, 172.65, 168.79, 168.67 (-CO-), 138.49, 138.13, 138.06, 137.88 (ipso-Ph von Benzyl, ipso-C von Phenylacetamid), 129.30 - 127.60 ((arom. CH), 87.01, 80.11, 79.93, 78.47, 78.33, 78.22, 78.10, 77.98, 77.22, 75.49, 75.42, 74.95, 74.82, 73.53, 73.4668.80, 68.50 (C-1 - C-6 von Glycosyl und CH₂-Ph), 64.40 (C-2 von Acetamid), 50.59, 48.40 (C-1 von Cyclohexyl), 32.85, 32.74 (C-2, C-6 von Cyclohexyl), 25.52, 24.75 (C-3, C-4, C-5 von Cyclohexyl), 22.75, 22.50 (CO-CH₃).
*A-3. 2-[(2,3,4,6-Tetra-O-benzyl-β-D-glucopyranosyl-carbonyl)-(4-methoxybenzyl)-amino]-N-cyclohexyl-2-phenyl-acetamid:*
   Aus Benzaldehyd (53 mg, 0.5 mmol), 4-Methoxybenzylamin (86 mg, 0.63 mmol), 2,6-Anhydro-3,4,5,7-tetra-O-benzyl-D-glycero-D-gulo-heptonsäure (355 mg, 0.63 mmol) und Cyclohexylisonitril (54 mg, 0.5 mmol). Säulenchromatographische Reinigung (Laufmittel Hexan-Essigester = 7:1). Ausbeute Diastereomer A: 117 mg (26 %); Diastereomer B: 104 mg (23 %). *Diastereomer A*: C₅₇ H₆₂ N₂ O₈ [903.1]. Sirup, [α]_{D} = + 53.7° (c = 0.38, Dichlormethan), R_{f} = 0.38 (Hexan-Essigester = 2:1). CI-MS: *m*/*z* 903 (*M* + H⁺); ¹³C-NMR (100.58 Mhz, CDCl₃): 169.36, 168.47, 168.36, 167.90 (-CO-), 159.02, 158.66 (C-4 von p-Methoxybenzyl), 138.95, 138.80, 138.59, 138.43, 138.29, 138.06, 138.03, 137.82 (ipso-Ph), 134.74, 134.65 (ipso-C von Phenylacetamid), 130.73 - 127.34 (arom. CH und C-1 von p-Methoxybenzyl), 114.21, 113.78 (C-3, C-5 von p-Methoxybenzyl), 86.93, 86.76, 80.10, 79.86, 79.17, 78.61, 77.86, 77.22, 76.58, 76.13, 75.74, 75.56, 75.02, 74.96, 74.80, 74.68, 73.44, 73.41, 69.23, 69.10 (C-1 - C-6 von Glycosyl und CH₂-Ph), 65.19, 63.61 (C-2 von Acetamid), 55.27, 55.18 (O-CH₃), 49.44, 48.47, 48.27, 45.77 (C-1 von Cyclohexyl und -CH₂-Ph-OMe), 32.72, 32.67, 32.46, 32.23 (C-2, C-6 von Cyclohexyl), 25.49, 25.29, 24.85, 24.77, 24.65 (C-3, C-4, C-5 von Cyclohexyl). *Diastereomer B*: C₅₇ H₆₂ N₂ O₈ [903.1]. Sirup, [α]_{D} = +16.4° (c = 0.61, Dichlormethan), R_{f} = 0.30 (Hexan-Essigester = 2:1). CI-MS: *m*/*z* 903 (*M* + H⁺); ¹³C-NMR (100.58 Mhz, CDCl₃): 169.50, 168.73, 168.04, 167.24 (-CO-), 158.78, 158.71 (C-4 von p-Methoxybenzyl), 138.61, 138.56, 138.46, 138.31, 138.25, 138.05, 137.84, 137.64 (ipso-Ph), 135.52, 134.91 (ipso-C von Phenylacetamid), 130.17 - 127.49 (arom. CH und C-1 von p-Methoxybenzyl), 113.81, 113.64 (C-3, C-5 von p-Methoxybenzyl), 86.88, 86.74, 79.84, 79.51, 79.30, 78.95, 78.46, 77.82, 77.74, 77.23, 76.13, 75.64, 75.58, 75.39, 75.03, 74.66, 73.45, 73.13, 68.99, 68.69 (C-1 - C-6 von Glycosyl und CH₂-Ph), 64.06, 63.85 (C-2 von Acetamid), 55.23, 55.19 (O-CH₃), 49.84, 49.81, 48.37, 47.96 (C-1 von Cyclohexyl und -CH₂-Ph-OMe), 32.73, 32.70, 32.41 (C-2, C-6 von Cyclohexyl), 25.47, 25.34, 25.08, 24.97, 24.65 (C-3, C-4, C-5 von Cyclohexyl).
*A-4. 2-[Acetyl-(4-methoxy-benzyl)-amino]-N-(2,3,4,6-tetra-O-benzyl-β-D-glucopyranosyl-methyl)-2-phenyl-acetamid:*
   Aus Benzäldehyd (53 mg, 0.5 mmol), 4-Methoxybenzylamin (86 mg, 0.63 mmol), Essigsäure (38 mg, 0.63 mmol), 2,6-Anhydro-3,4,5,7-tetra-O-benzyl-1-desoxy-1-isocyano-D-glycero-D-gulo-heptitol (282 mg, 0.5 mmol) und Zinkchlorid (30 mg). Säulenchromatographische Reinigung (Laufmittel Hexan-Essigester = 3:1). Ausbeute 204 mg (48 %). C₅₃ H₅₆ N₂ O₈ [849.1]. Sirup, R_{f} = 0.11 (Hexan-Essigester = 1:1). CI-MS: *m*/*z* 849 (*M* + H⁺); ¹³C-NMR (100.58 Mhz, CDCl₃): 169.33, 168.87 (-CO-), 158.53 (C-4 von p-Methoxybenzyl), 138.22, 138.08, 137.90, 137.78 (ipso-Ph), 134.99 (ipso-C von Phenylacetamid), 130.10 - 127.49 (arom. CH und C-1 von p-Methoxybenzyl), 113.78 (C-3, C-5 von p-Methoxybenzyl), 86.87, 78.74, 78.68, 78.10, 75.51, 75.17, 75.09, 75.00, 73.49, 68.78 (C-1 - C-6 von Glycosyl und CH₂-Ph), 63.74 (C-2 von Acetamid), 55.21 (O-CH₃), 29.71 (Glycosyl-CH₂-NHCO-), 22.50 (CO-CH₃).
*A-5. 2-[Acetyl-(2,3,4,6-tetra-O-benzyl-β-D-glucopyranosyl-methyl) amino]-N-cyclohexyl-2-(2,3,4,6-tetra-O-benzyl-β-D-glucopyranosyl)-acetamid:*
   Aus 2,6-Anhydro-3,4,5,7-tetra-O-benzyl-D-glycero-D-gulo-heptose (277 mg, 0.5 mmol), 1-Amino-2,6-anhydro-3,4,5,7-tetra-O-benzyl-1-desoxy-D-glycero-D-gulo-heptitol (349 mg, 0.63 mmol), Essigsäure (38 mg, 0.63 mmol), und Cyclohexylisonitril (54 mg, 0.5 mmol). Säulenchromatographische Reinigung (Laufmittel Hexan-Essigester = 2:1). Ausbeute Diastereomer A 194 mg (31 %), Diastereomer B 173 mg (27 %). *Diastereomer A*: C₇₉ H₈₈ N₂ O₁₂ [1257.6]. Sirup, [α]_{D} = -10.4° (c = 0.24, Dichlormethan), R_{f} = 0.23 (Hexan-Essigester = 2:1). CI-MS: *m*/*z* 1257 (*M* + H⁺). *Diastereomer B*: C₇₉ H₈₈ N₂ O₁₂ [1257.6]. Sirup, [α]_{D} = +13.2° (c = 0.25, Dichlormethan), R_{f} = 0.05 (Hexan-Essigester = 2:1). CI-MS: *m*/*z* 1257 (*M* + H⁺).
*A-6. 2-[(2,3,4,6-tetra-O-benzyl-β-D-glucopyranosyl-carbonyl)-4-methoxybenzylamino]-N-cyclohexyl-2-(2,3,4,6-tetra-O-benzyl-β-D-glucopyranosyl)-acetamid:*
   Aus 2,6-Anhydro-3,4,5,7-tetra-O-benzyl-D-glycero-D-gulo-heptose (277 mg, 0.50 mmol), p-Methoxybenzylamin (86 mg, 0.63 mmol), 2,6-Anhydro-3,4,5,7-tetra-O-benzyl-D-glycero-D-gulo-heptonsäure (355 mg, 0.63 mmol) und Cyclohexylisonitril (54 mg, 0.5 mmol). Säulenchromatographische Reinigung (Laufmittel Hexan-Essigester = 5:1). Ausbeute Diastereomer A 157 mg (23 %), Diastereomer B 325 mg (48 %). *Diastereomer A*: C₈₅ H₉₂ N₂ O₁₃ [1349.7]. Sirup, [α]_{D} = +42.0° (c = 0.56, Dichlormethan), R_{f} = 0.48 (Hexan-Essigester = 2:1). CI-MS: *m*/*z* 1349 (*M* + H⁺); ¹³C-NMR (100.58 Mhz, CDCl₃): 170.09, 168,43, 166.49, 166.06 (-CO-), 158.55, 158.21 (C-4 von p-Methoxybenzyl), 138.65, 138.54, 138.43, 138.40, 138.34, 138.07, 138.04, 137.98, 137.70 (ipso-Ph), 129.88 - 126.72 (arom. CH und C-1 von p-Methoxybenzyl), 113.89, 113.65 (C-3, C-5 von p-Methoxybenzyl), 87.00, 86.76, 86.67, 80.49, 79.88, 79.79, 79.67, 79.18, 79.06, 78.93, 78.52, 77.87, 77.22, 76.70, 75.64, 75.57, 75.47, 75.33, 75.25, 75.11, 74.95, 74.80, 74.57, 74.35, 73.35, 73.22, 69.15, 68.53, 68.25, 67.67 (C-1 - C-6 von Glycosyl und CH₂-Ph), 59.48, 57.16 (C-2 von Acetamid), 55.20 (O-CH₃), 48.57, 48.25, 47.77 (C-1 von Cyclohexyl und -CH₂-Ph-OMe), 32.86, 32.70, 32.55 (C-2, C-6 von Cyclohexyl), 25.39, 25.27, 24.77 (C-3, C-4, C-5 von Cyclohexyl). *Diastereomer B*: C₈₅ H₉₂ N₂ O₁₃ [1349.7]. Sirup, [α]_{D} = +11.0° (c = 0.74, Dichlormethan), R_{f} = 0.38 (Hexan-Essigester = 2:1). CI-MS: *m*/*z* 1349 (*M* + H⁺); ¹³C-NMR (100.58 Mhz, CDCl₃): 170.72, 166.90 (-CO-), 158.71 (C-4 von p-Methoxybenzyl), 138.85, 138.65, 138.43, 138.39, 138.17, 138.09, 137.94, (ipso-Ph), 131.01 - 126.65 (arom. CH und C-1 von p-Methoxybenzyl), 113.75 (C-3, C-5 von p-Methoxybenzyl), 87.27, 86.80, 80.14, 78.84, 78.31, 78.21, 77.75, 77.22, 75.66, 75.50, 74.99, 74.80, 74.71, 74.62, 74.02, 73.19, 73.01, 69.26 (C-1 - C-6 von Glycosyl und CH₂-Ph), 55.27 (O-CH₃), 48.77 (C-2 von Acetamid), 33.04, 32.60 (C-1, C-2, C-6 von Cyclohexyl), 25.48, 24.96 (C-3, C-4, C-5 von Cyclohexyl).
*A-7. 2-[(2,3,4,6-tetra-O-benzyl-β-D-glucopyranosyl-carbonyl)-(2,3,4,6-tetra-O-benzyl-β-D-glucopyranosyl-methyl)-amino]-N-cyclohexyl-2-phenyl-acetamid:*
   Aus Benzaldehyd (53 mg, 0.50 mmol), 1-Amino-2,6-anhydro-3,4,5,7-tetra-O-benzyl-1-desoxy-D-glycero-D-gulo-heptitol (349 mg, 0.63 mmol), 2,6-Anhydro-3,4,5,7-tetra-O-benzyl-D-glycero-D-gulo-heptonsäure (355 mg, 0.63 mmol) und Cyclohexylisonitril (54 mg, 0.5 mmol). Säulenchromatographische Reinigung (Laufmittel Petrolether-Essigester = 5:1). Ausbeute 247 mg (37 %). C₈₄ H₉₀ N₂ O₁₂ [1319.7]. Sirup, [α]_{D} = +9.6° (c = 0.35, Dichlormethan), R_{f} = 0.27 (Hexan-Essigester = 2:1). CI-MS: *m*/*z* 1319 (*M* + H⁺).
*A-8. 2-[(2,3,4,6-tetra-O-benzyl-β-D-glucopyranosyl-carbonyl)-(4-methoxybenzyl)-amino]-N-(2,3,4,6-tetra-O-benzyl-β-D-glucopyranosyl-methyl)-2-phenyl*-*acetamid:*
   Aus Benzaldehyd (53 mg, 0.50 mmol), 4-Methoxybenzylamin ((86 mg, 0.63 mmol), 2,6-Anhydro-3,4,5,7-tetra-O-benzyl-D-glycero-D-gulo-heptonsäure (355 mg, 0.63 mmol), 2,6-Anhydro-3,4,5,7-tetra-O-benzyl-1-desoxy-1-isocyano-D-glycero-D-gulo-heptitol (281 mg, 0.5 mmol) und Zinkchlorid (89 mg, 0.65 mmol). Säulenchromatographische Reinigung (Laufmittel Hexan-Essigester = 3:1). Ausbeute 239 mg (35 %). C₈₆ H₈₈ N₂ O₁₃ [1357.7]. Sirup, R_{f} = 0.26 (Hexan-Essigester = 2:1). FAB-MS: *m*/*z* 1379 (*M* + Na⁺) *m*/*z* 1355 (*M* - H⁺).
*A-9. 2-[Acetyl-(2,3,4,6-tetra-O-benzyl-β-D-glucopyranosyl-methyl)-amino]-N-(2,3,4,6-tetra-O-benzyl-β-D-glucopyranosyl-methyl)-2-phenyl-acetamid:*
   Aus Benzaldehyd (53 mg; 0.50 mmol), 1-Amino-2,6-anhydro-3,4,5,7-tetra-O-benzyl-1-desoxy-D-glycero-D-gulo-heptitol (349 mg, 0.63 mmol), Essigsäure (38 mg, 0.63 mmol), 2,6-Anhydro-3,4,5,7-tetra-O-benzyl-1-desoxy-1-isocyano-D-glycero-D-gulo-heptitol (282 mg, 0.5 mmol) und Zinkchlorid (89 mg, 0.65 mmol). Säulenchromatographische Reinigung (Laufmittel Hexan-Essigester = 3:1). Ausbeute 284 mg (45 %). C₈₀ H₈₄ N₂ O₁₂ [1265.6]. Sirup, R_{f} = 0.24 (Hexan-Essigester = 2:1). FAB-MS: *m*/*z* 1265 (*M* + H⁺), *m*/*z* 1263 (*M* - H⁺).
*A-10. 2-[Acetyl-(4-methoxy-benzyl)-amino]-N-(2,3,4,6-tetra-O-benzyl-β-D-glucopyranosyl-methyl)-2-(2,3,4,6-tetra-O-benzyl-β-D-glucopyranosyl)-acetamid:*
   Aus 2,6-Anhydro-3,4,5,7-tetra-O-benzyl-D-glycero-D-gulo-heptose (276 mg, 0.50 mmol), 4-Methoxybenzylamin (86 mg, 0.63 mmol), Essigsäure (38 mg, 0.63 mmol), 2,6-Anhydro-3,4,5,7-tetra-O-benzyl-1-desoxy-1-isocyano-D-glycero-D-gulo-heptitol (281 mg, 0.5 mmol) und Zinkchlorid (89 mg, 0.65 mmol). Säulenchromatographische Reinigung (Laufmittel Hexan-Essigester = 3:1). Ausbeute 223 mg (34 %). C₈₁ H₈₆ N₂ O₁₃ [1295.6]. Sirup, R_{f} = 0.26 (Hexan-Essigester = 2:1). FAB-MS: *m*/*z* 1295 (*M* + H⁺), *m*/*z* 1293 (*M* - H⁺).
*A-11. 2-[(2,3,4,6-tetra-O-benzyl-β-D-glucopyranosyl-carbonyl)-(2,3,4,6-tetra-O-benzyl-β-D-glucopyranosyl-methyl)-amino]-N-cyclohexyl-2-(2,3,4,6-tetra-O-benzyl-β-D-glucopyranosyl)-acetamid:*
   Aus 2,6-Anhydro-3,4,5,7-tetra-O-benzyl-D-glycero-D-gulo-heptose (277 mg, 0.5 mmol), 1-Amino-2,6-anhydro-3,4,5,7-tetra-O-benzyl-1-desoxy-D-glycero-D-gulo-heptitol (349 mg, 0.63 mmol), 2,6-Anhydro-3,4,5,7-tetra-O-benzyl-D-glycero-D-gulo-heptonsäure (355 mg, 0.63 mmol) und Cyclohexylisonitril (54 mg, 0.5 mmol). Säulenchromatographische Reinigung (Laufmittel Hexan-Essigester = 7:1). Ausbeute 113 mg (13 %). C₁₁₂ H₁₂₀ N₂ O₁₇ [1766.2]. Sirup. Diastereomer A: R_{f} = 0.51, Diastereomer B: R_{f} = 0.47 (Hexan-Essigester = 2:1). FAB-MS: *m*/*z* 1787 (*M* + Na⁺), *m*/*z* 1763 (*M* - H⁺).
*A-12. 2-[(2,3,4,6-tetra-O-benzyl-β-D-glucopyranosyl-carbonyl)-(4-methoxybenzyl)-amino]-N-(2,3,4,6-tetra-O-benzyl-β-D-glucopyranosyl-methyl)-2-(2,3,4,6-tetra-O-benzyl-β-D-glucopyranosyl)-acetamid:*
   Aus 2,6-Anhydro-3,4,5,7-tetra-O-benzyl-D-glycero-D-gulo-heptose (277 mg, 0.5 mmol), 4-Methoxybenzylamin (86 mg, 0.63 mmol), 2,6-Anhydro-3,4,5,7-tetra-O-benzyl-D-glycero-D-gulo-heptonsäure (355 mg, 0.63 mmol), 2,6-Anhydro-3,4,5,7-tetra-O-benzyl-1-desoxy-1-isocyano-D-glycero-D-gulo-heptitol (282 mg, 0.5 mmol) und Zinkchlorid (89 mg, 0.65 mmol). Säulenchromatographische Reinigung (Laufmittel Hexan-Essigester = 7:1). Ausbeute 111 mg (12 %). C₁₁₄ H₁₁₈ N₂ O₁₈ [1804.2]. Sirup. R_{f} = 0.48 (Hexan-Essigester = 2:1). FAB-MS: *m*/*z* 1803 (*M* + H⁺), *m*/*z* 1801 (*M* - H⁺).
*A-13. 2-[Acetyl-(2,3,4,6-tetra-O-benzyl-β-D-glucopyranosyl-methyl)-amino]-N-(2,3,4,6-tetra-O-benzyl-β-D-glucopyranosyl-methyl)-2-(2,3,4,6-tetra-O-benzyl-β-D-glucopyranosyl)-acetamid:*
   Aus 2,6-Anhydro-3,4,5,7-tetra-O-benzyl-D-glycero-D-gulo-heptose (277 mg, 0.5 mmol), 1-Amino-2,6-anhydro-3,4,5,7-tetra-O-benzyl-1-desoxy-D-glycero-D-gulo-heptitol (349 mg, 0.63 mmol), Essigsäure (38 mg, 0.63 mmol), 2,6-Anhydro-3,4,5,7-tetra-O-benzyl-1-desoxy-1-isocyano-D-glycero-D-gulo-heptitol (282 mg, 0.5 mmol) und Zinkchlorid (89 mg, 0.65 mmol). Säulenchromatographische Reinigung (Laufmittel Hexan-Essigester = 7:1). Ausbeute 174 mg (20 %). C₁₀₈ H₁₁₄ N₂ O₁₇ [1712.1]. Sirup. R_{f} = 0.44 (Hexan-Essigester = 2:1). FAB-MS: *m*/*z* 1711 (*M* + H⁺), *m*/*z* 1709 (*M* - H⁺).
*A-14. 2-[(2,3,4,6-Tetra-O-benzyl-β-D-glucopyranosyl-carbonyl)-(2,3,4,6-tetra-O-benzyl-β-D-glucopyranosyl-methyl)-amino]-N-(2,3,4,6-tetra-O-benzyl-β-D-glucopyranosyl-methyl)-2-phenyl-acetamid:*
   Aus Benzaldehyd (53 mg, 0.5 mmol), 1-Amino-2,6-anhydro-3,4,5,7-tetra-O-benzyl-1-desoxy-D-glycero-D-gulo-heptitol (349 mg, 0.63 mmol), 2,6-Anhydro-3,4,5,7-tetra-O-benzyl-D-glycero-D-gulo-heptonsäure (355 mg, 0.63 mmol), 2,6-Anhydro-3,4,5,7-tetra-O-benzyl-1-desoxy-1-isocyano-D-glycero-D-gulo-heptitol (282 mg, 0.5 mmol) und Zinkchlorid (89 mg, 0.65 mmol). Säulenchromatographische Reinigung (Laufmittel Hexan-Essigester = 7:1). Ausbeute 160 mg (18 %). C₁₁₃ H₁₁₆ N₂ O₁₇ [1774.2]. Sirup. R_{f} = 0.51 (Hexan-Essigester = 2:1). FAB-MS: *m*/*z* 1795 (*M* + Na⁺), *m*/*z* 1771 (*M*- H⁺).
*A-15. 2-[(2,3,4,6-Tetra-O-benzyl-β-D-glucopyranosyl-carbonyl)-(2,3,4,6-tetra-O-benzyl-β-D-glucopyranosyl-methyl)-amino]-N-(2,3,4,6-tetra-O-benzyl-β-D-glucopyranosyl-methyl)-2-(2,3,4,6-tetra-O-benzyl-β-D-glucopyranosyl)-acetamid:*
   Aus 2,6-Anhydro-3,4,5,7-tetra-O-benzyl-D-glycero-D-gulo-heptose (277 mg, 0.5 mmol), 1-Amino-2,6-anhydro-3,4,5,7-tetra-O-benzyl-1-desoxy-D-glycero-D-gulo-heptitol (349 mg, 0.63 mmol), 2,6-Anhydro-3,4,5,7-tetra-O-benzyl-D-glycero-D-gulo-heptonsäure (355 mg, 0.63 mmol), 2,6-Anhydro-3,4,5,7-tetra-O-benzyl-1-desoxy-1-isocyano-D-glycero-D-gulo-heptitol (282 mg, 0.5 mmol) und Zinkchlorid (89 mg, 0.65 mmol). Säulenchromatographische Reinigung (Laufmittel Hexan-Essigester = 10:1). Ausbeute 95 mg (9 %). C₁₄₁ H₁₄₆ N₂ O₂₂ [2220.7]. Sirup. R_{f} = 0.46 (Hexan-Essigester = 4:1). FAB-MS: *m*/*z* 2241 (*M* + Na⁺), *m*/*z* 2217 (*M* - H⁺).

Entsprechend der allgemeinen Vorschrift A wurden die Verbindungen der allgemeinen Formel (I), wobei X = NH bedeutet, hergestellt:

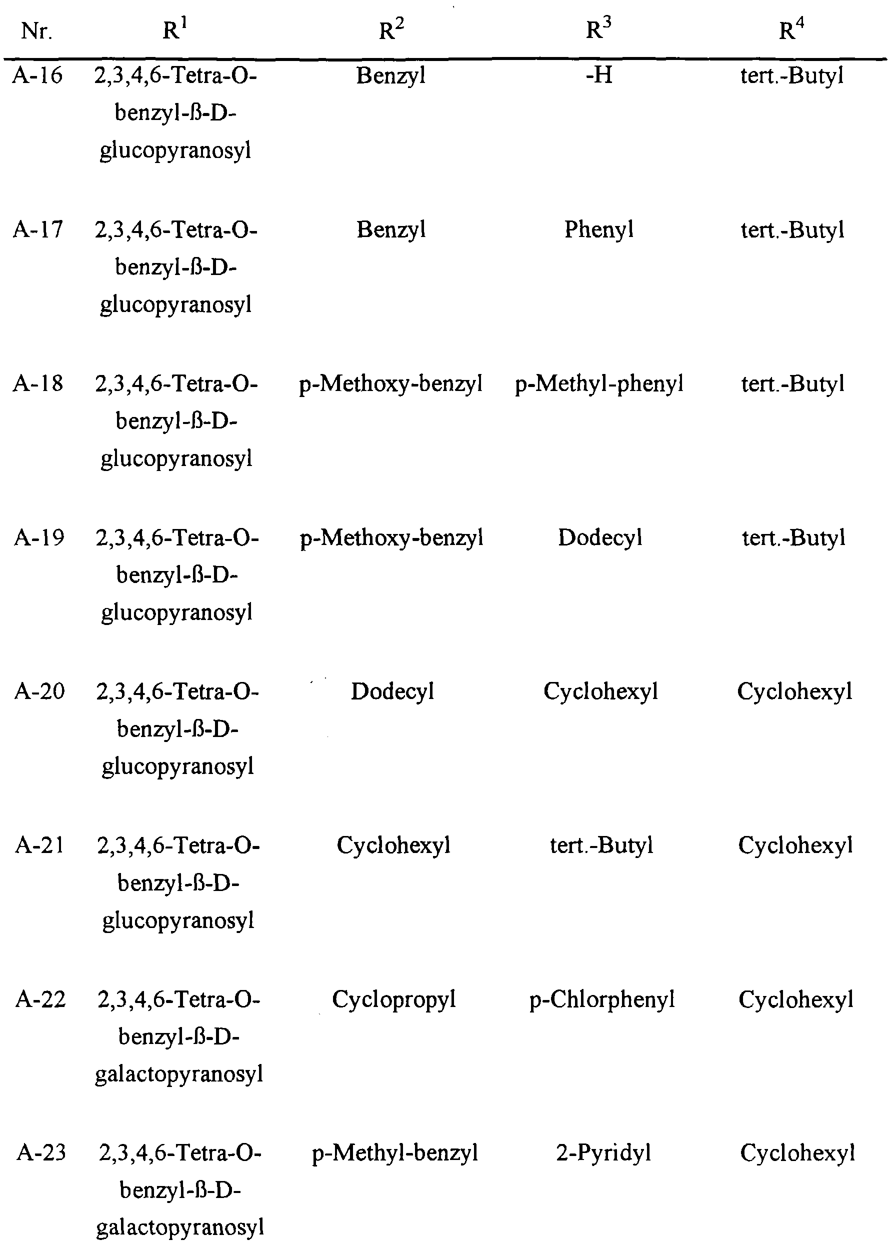

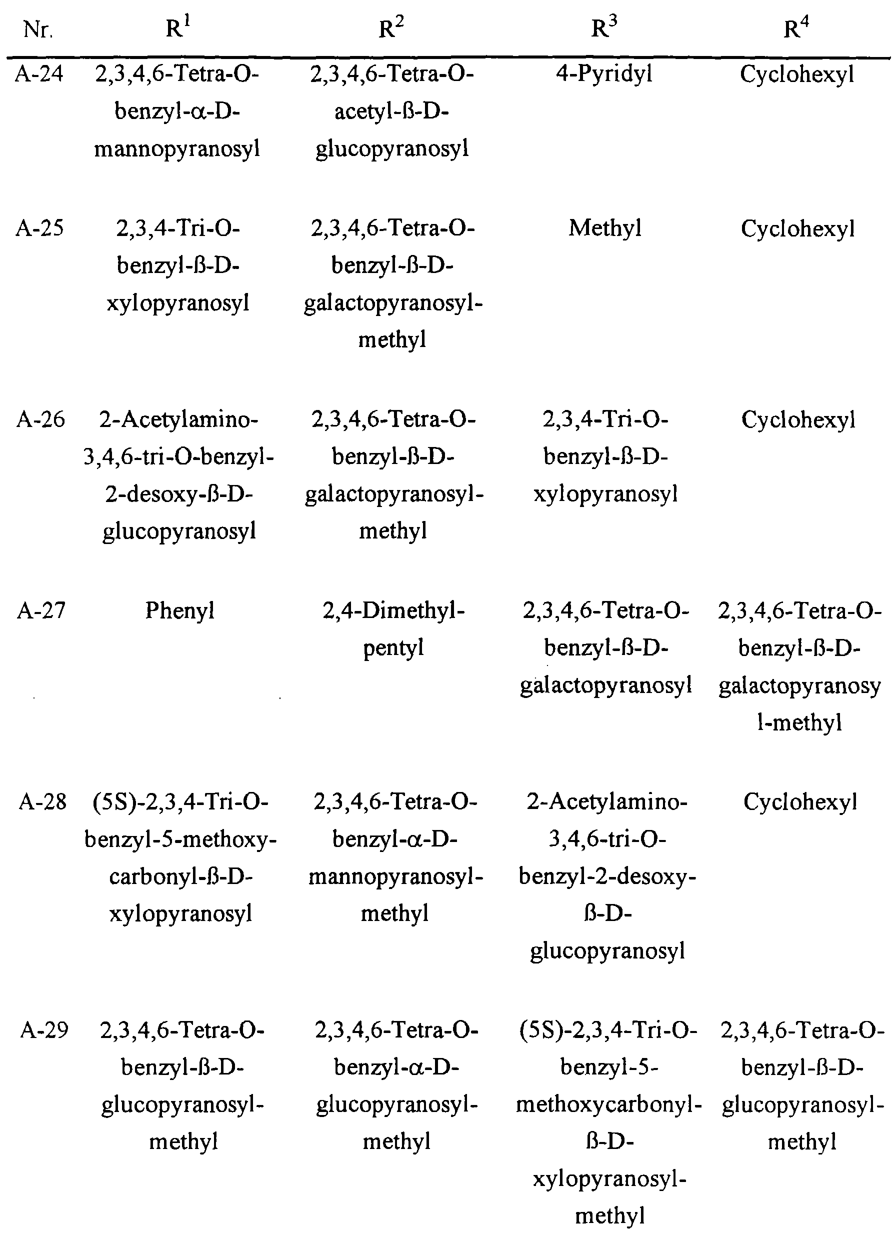

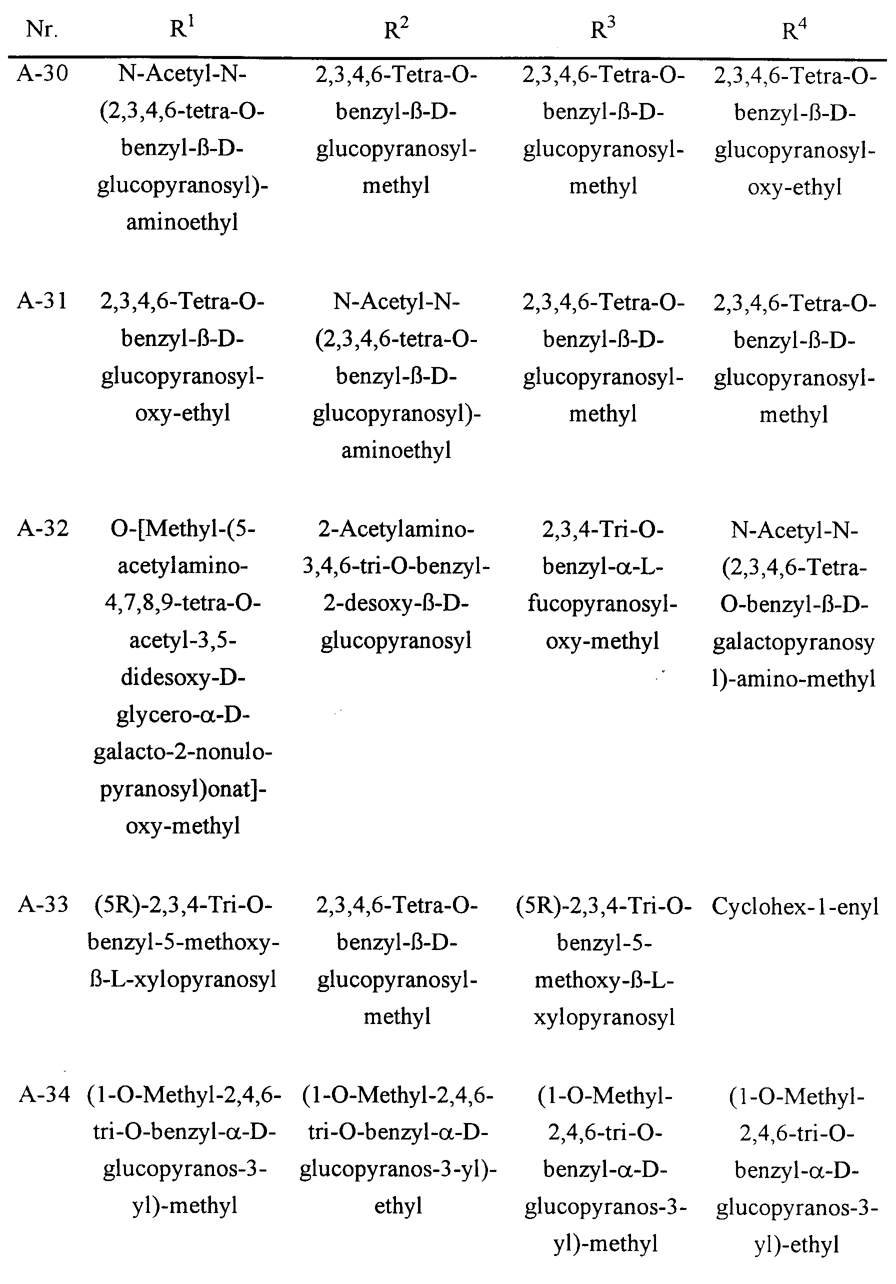

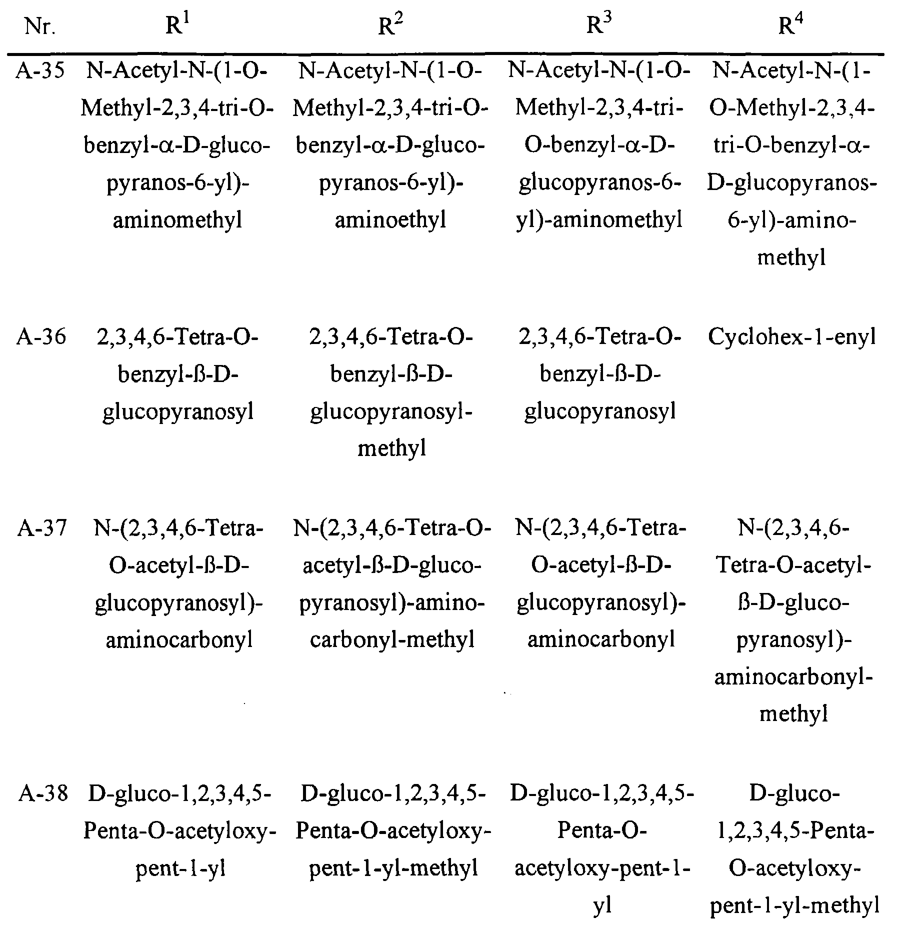

*A-39. 2-(N-Acetyl-N-4-methoxybenzyl-amino)-3-(2,3,4,6-tetra-O-benzyl-β-D-gluco-pyranosyloxy)-N-cyclohexyl-propionamid:* Aus 2-(2,3,4,6-Tetra-O-benzyl-β-D-glucopyranosyl-oxy)-acetaldehyd (291 mg, 0.5 mmol), 4-Methoxybenzylamin (86 mg, 0.63 mmol), Essigsäure (38 mg, 0.63 mmol), Cyclohexylisonitril (54 mg, 0.5 mmol) und Zinkchlorid (1 mg). Säulenchromatographische Reinigung (Laufmittel Hexan-Essigester = 3 : 1). Diastereomerengemisch ca 1 : 1. Ausbeute 230 mg (53 %). C₅₃ H₆₂ N₂ O₉ [871.1]. Sirup. CI-MS: *m*/*z* 871 (*M* + H⁺).
*A-40. 2-[Acetyl-(2,3,4,6-Tetra-O-benzyl-β-D-glucopyranosyl-oxy-ethyl)-amino]-N cyclohexyl-2-phenyl-acetamid:* Aus Benzaldehyd (53 mg, 0.5 mmol), 2-(2,3,4,6-Tetra-O-benzyl-β-D-glucopyranosyl-oxy)-ethylamin (367 mg, 0.63 mmol), Essigsäure (38 mg, 0.63 mmol), Cyclohexylisonitril (54 mg, 0.5 mmol) und Zinkchlorid (1 mg). Säulenchromatographische Reinigung (Laufmittel Petrolether-Essigester = 2 : 1). Ausbeute 256 mg (61 %). Es wurden zwei nicht trennbare Diasteromere erhalten. C₅₂ H₆₀ N₂ O₈ [841.1]. Sirup, [α]_{D} = 4.2 ° (c = 0.28, Dichlormethan), R_{f} = 0.28 (Hexan-Essigester = 1 : 1). CI-MS: *m*/*z* 841 (*M* + H⁺).
*A-41. 2-[N-(2,3,4,6-Tetra-O-benzyl-β-D-glucopyranosyl-oxy-methylcarbonyl)-N-(4-methoxybenzyl)-amino]-N-cyclohexyl-2-phenyl-acetamid:* Aus Benzaldehyd (53 mg, 0.5 mmol), 4-Methoxybenzylamin (86 mg, 0.63 mmol), 2-(2,3,4,6-Tetra-O-benzyl-β-D-glucopyranosyl-oxy)-essigsäure (377 mg, 0.63 mmol) und Cyclohexylisonitril (54 mg, 0.5 mmol). Säulenchromatographische Reinigung (Laufmittel Hexan-Essigester = 3 : 1). Diastereomerenmischung. Ausbeute 219 mg (47 %). C₅₈ H₆₄ N₂ O₉ [933.2]. Sirup, R_{f} = 0.55 (Hexan-Essigester = 1 : 1). CI-MS: *m*/*z* 933 (*M* + H⁺).
*A-42. 2-[Acetyl-(4-methoxybenzyl)-amino]-N-(2,3,4,6-tetra-O-benzyl-β-D-gluco-pyranosyl-oxy-ethyl)-2-phenyl-acetamid:* Aus Benzaldehyd (53 mg, 0.5 mmol), 4-Methoxybenzylamin (86 mg, 0.63 mmol), Essigsäure (38 mg, 0.63 mmol), 2-(2,3,4,6-Tetra-O-benzyl-β-D-glucopyranosyl-oxy)-ethylisonitril (296 mg, 0.5 mmol) und Zinkchlorid (1 mg). Säulenchromatographische Reinigung (Laufmittel Hexan-Essigester = 2 : 1). Diastereomerenmischung. Ausbeute 241 mg (55 %). C₅₄ H₅₈ N₂ O₉ [879.1]. Sirup, R_{f} = 0.14 (Hexan-Essigester = 1 : 1). CI-MS: *m*/*z* 879 (*M* + H⁺).
*A-43. 2-[N-(2,3,4,6-Tetra-O-benzyl-β-D-glucopyranosyl-oxy-methylcarbonyl)-N-(2,3,4,6-tetra-O-benzyl-β-D-glucopyranosyl-oxy-ethyl)-amino]-3-(2,3,4,6-tetra-O-benzyl-β-D-glucopyranosyloxy)-N-(2,3,4,6-tetra-O-benzyl-β-D-glucopyranosyl-oxy-ethyl)-propionamid:* Aus 2-(2,3,4,6-Tetra-O-benzyl-β-D-glucopyranosyl-oxy)-acetaldehyd (29.1 mg, 0.05 mmol), 2-(2,3,4,6-Tetra-O-benzyl-β-D-glucopyranosyl-oxy)-ethylamin (36.7 mg, 0.063 mmol), 2-(2,3,4,6-Tetra-O-benzyl-β-D-glucopyranosyl-oxy)-essigsäure (37.7 mg, 0.063 mmol), 2-(2,3,4,6-Tetra-O-benzyl- β-D-glucopyranosyl-oxy)-ethylisonitril (29.6 mg, 0.05 mmol) und Zinkchlorid (1 mg). Säulenchromatographische Reinigung (Laufmittel Toluol-Essigester = 10 : 1). Diastereomerenmischung. Ausbeute 56 mg (48 %). C₁₄₅ H₁₅₄ N₂ O₂₆ [2340.8]. Sirup, R_{f} = 0.36 (Hexan-Essigester = 2 : 1). MALDI-MS: *m*/*z* 2361.1 (*M* + Na⁺).
*B-1. 2-[(2,3,4,6-Tetra-O-benzyl-β-D-glucopyranosyl-carbonyl)-(2,3,4,6-tetra-O-benzyl-β-D-glucopyranosyl-methyl)-amino]- 2-(2,3,4,6-tetra-O-benzyl-β-D-glucopyranosyl)-essigsäure.:*
   2-[(2,3,4,6-tetra-O-benzyl-β-D-glucopyranosyl-carbonyl)-(2,3,4,6-tetra-O-benzyl-β-D-glucopyranosyl-methyl)-amino]-N-(cyclohex-1-enyl)-2-(2,3,4,6-tetra-O-benzyl-β-D-glucopyranosyl)-acetamid (Beispiel A-36; 176 mg, 0.1 mmol) wird Tetrahydrofuran (2.0 ml) gelöst und mit konzentrierter Salzsäure (0.1 ml) versetzt. Nach 16 h wird mit festem Natriumhydrogencarbonat neutralisiert und filtriert und eingeengt. Der Rückstand wird durch Flash-Chromatographie über Kieselgel gereinigt (Laufmittel Hexan-Essigester = 2:1). Ausbeute 155 mg (92 %). C₁₀₆ H₁₀₉ N O₁₈ [1685.1]. Sirup. Rf = 0.23 (Hexan-Essigester = 2 : 1). FAB-MS: *m*/*z* 1706 (*M* + Na⁺), *m*/*z* 1682 (*M* - H⁺).
*C-1. 2-[(2,3,4,6-Tetra-O-benzyl-β-D-glucopyranosyl-carbonyl)-(2,3,4,6-tetra-O-benzyl-β-D-glucopyranosyl-methyl)-amino]-N-(2,3,4,6-tetra-O-benzyl-β-D-galactopyranosyl-methyl)-2-(2,3,4,6-tetra-O-benzyl-β-D-glucopyranosyl)-acetamid:*
   Die Mischung aus der Verbindung aus Beispiel B-1 (100 mg, 0.06 mmol), N-Hydroxysuccinimid (13.7 mg, 0.12 mmol) und Dicyclohexylcarbodiimid (18.2 mg, 0.09 mmol) in Tetrahydrofuran (1 ml) wird 2 h bei Raumtemperatur gerührt und mit 2,3,4,6-Tetra-O-benzyl-β-D-galactopyranosyl-methyl-amin (33 mg, 0.09 mmol) versetzt. Nach 16 h wird im Vakuum eingeengt. Säulenchromatographische Reinigung (Laufmittel Hexan-Essigester = 10:1). Ausbeute 108 mg (82 %). C₁₄₁ H₁₄₆ N₂ O₂₂ [2220.7]. Sirup. R_{f} = 0.46 (Hexan-Essigester = 4:1). FAB-MS: *m*/*z* 2241 (*M* + Na⁺) *m*/*z* 2217 (*M* - H⁺).
*D. Allgemeine Vorschrift zur Spaltung der Acerylester-Schutzgruppen:*
   Die unter A genannte Verbindung (0.1 mmol) wird in Tetrahydrofuran (1.0 ml) und Methanol (1.0 ml) gelöst und mit 1 N Natriummethanolat (0.05 ml) versetzt. Nach 16 h wird mit Kationenaustauscher IR120 (H+-Form) neutralisiert, filtriert und eingeengt. Der Rückstand wird gefriergetrocknet.
*E. Allgemeine Vorschrift zur Spaltung der Benzylether-Schutzgruppen:*
   Die unter A genannte oder gemäß allgemeiner Vorschrift D deacetylierte Verbindung (0.1 mmol) wird in Tetrahydrofuran (1.0 ml), Methanol (1.0 ml), Wasser (0.2 ml) und Eisessig (0.03 ml) gelöst, mit Palladium-Kohle (10 %-ig; 15 mg) versetzt und bei Normaldruck hydriert. Der Katalysator wird abfiltriert und mit Wasser (0.5 ml) gewaschen. Die Filtrate werden vereinigt und eingeengt. Der Rückstand wird gefriergetrocknet.

Auf diese Weise wurden aus den Verbindungen A-1 bis A-38 die folgenden Verbindungen E-1 bis E-38 der Formel (I), wobei X = NH bedeutet, erhalten:

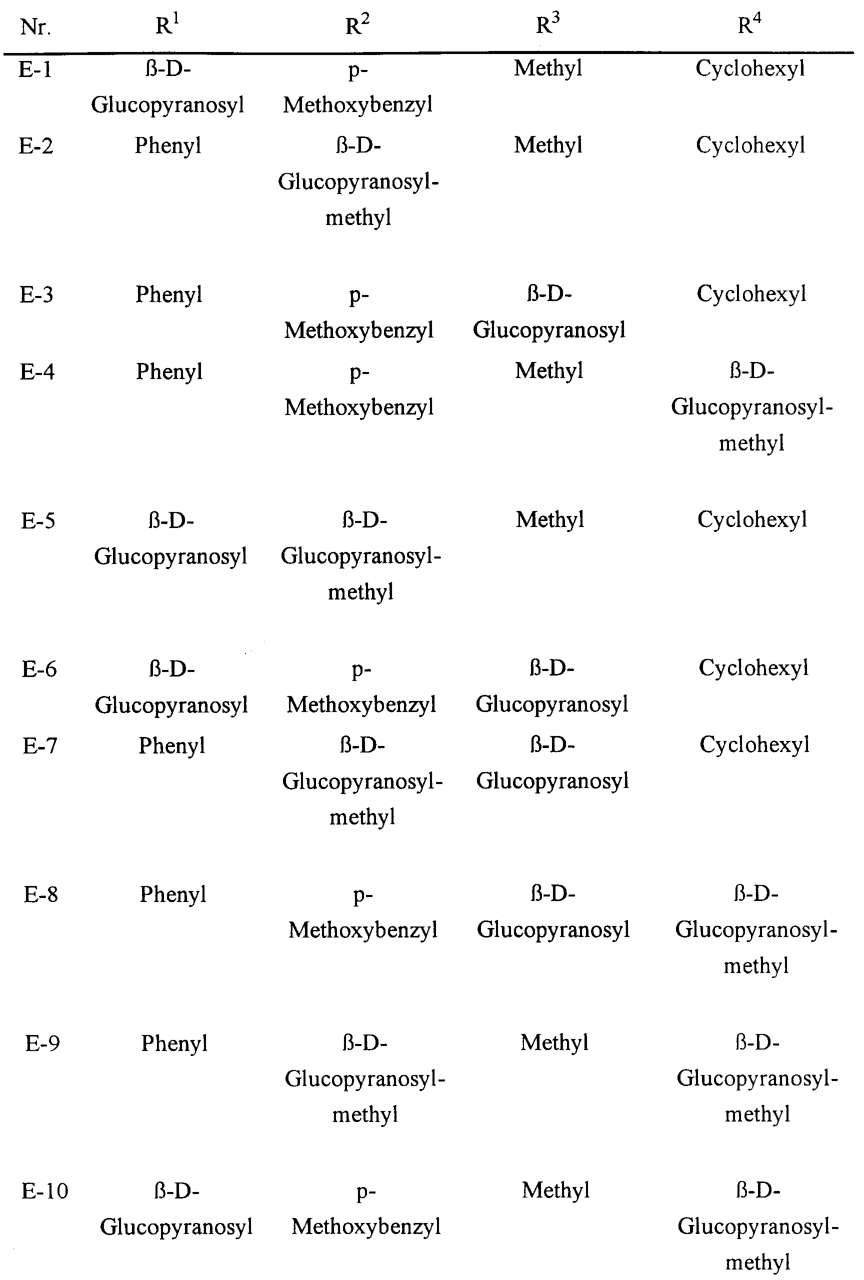

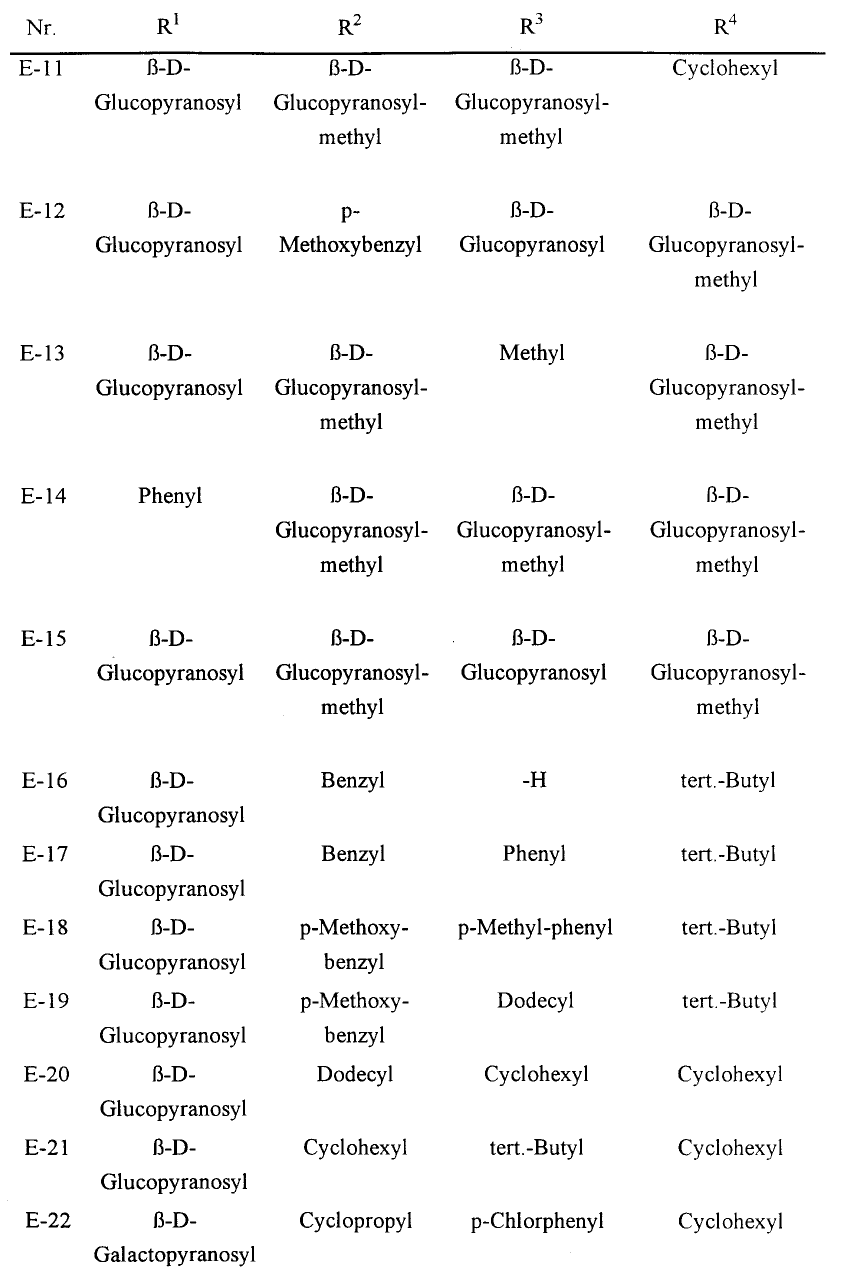

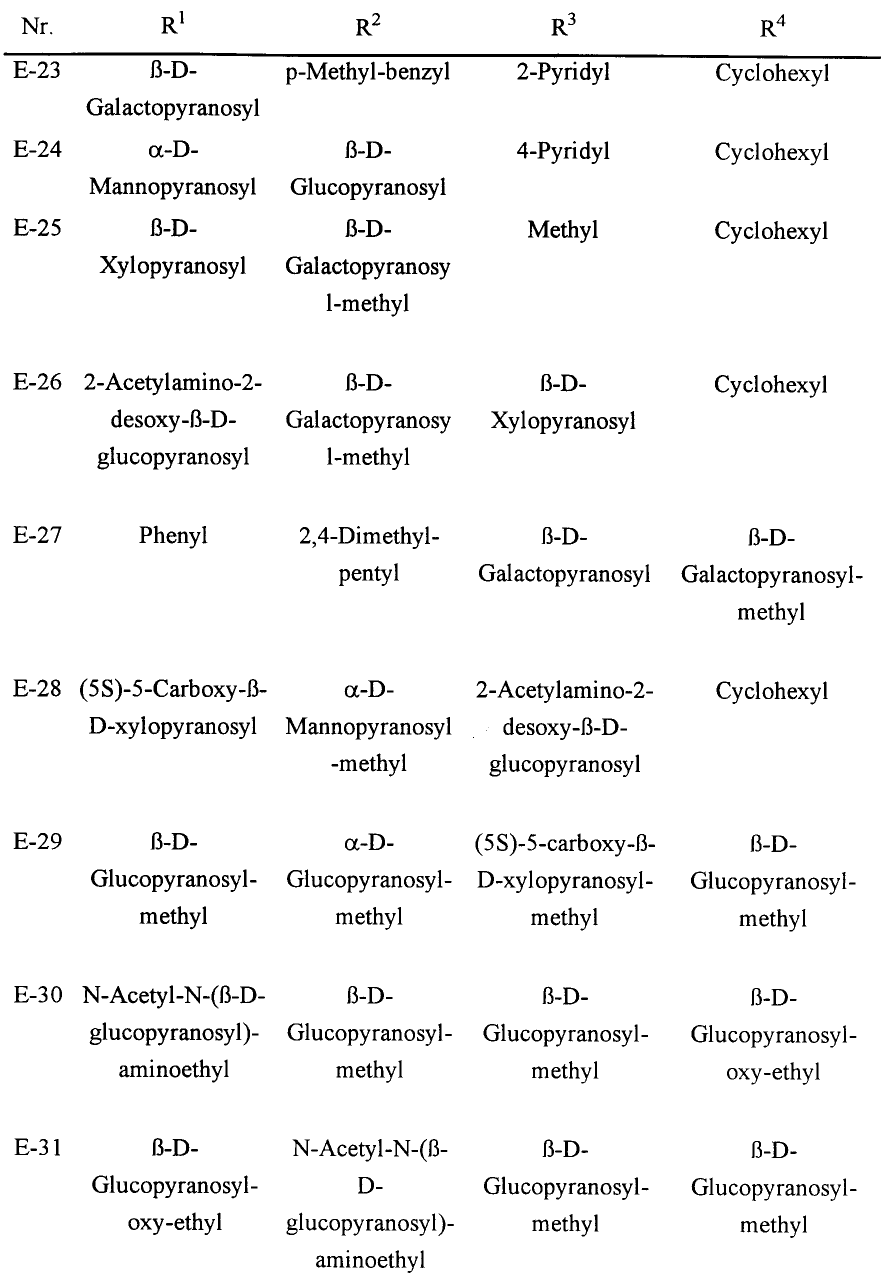

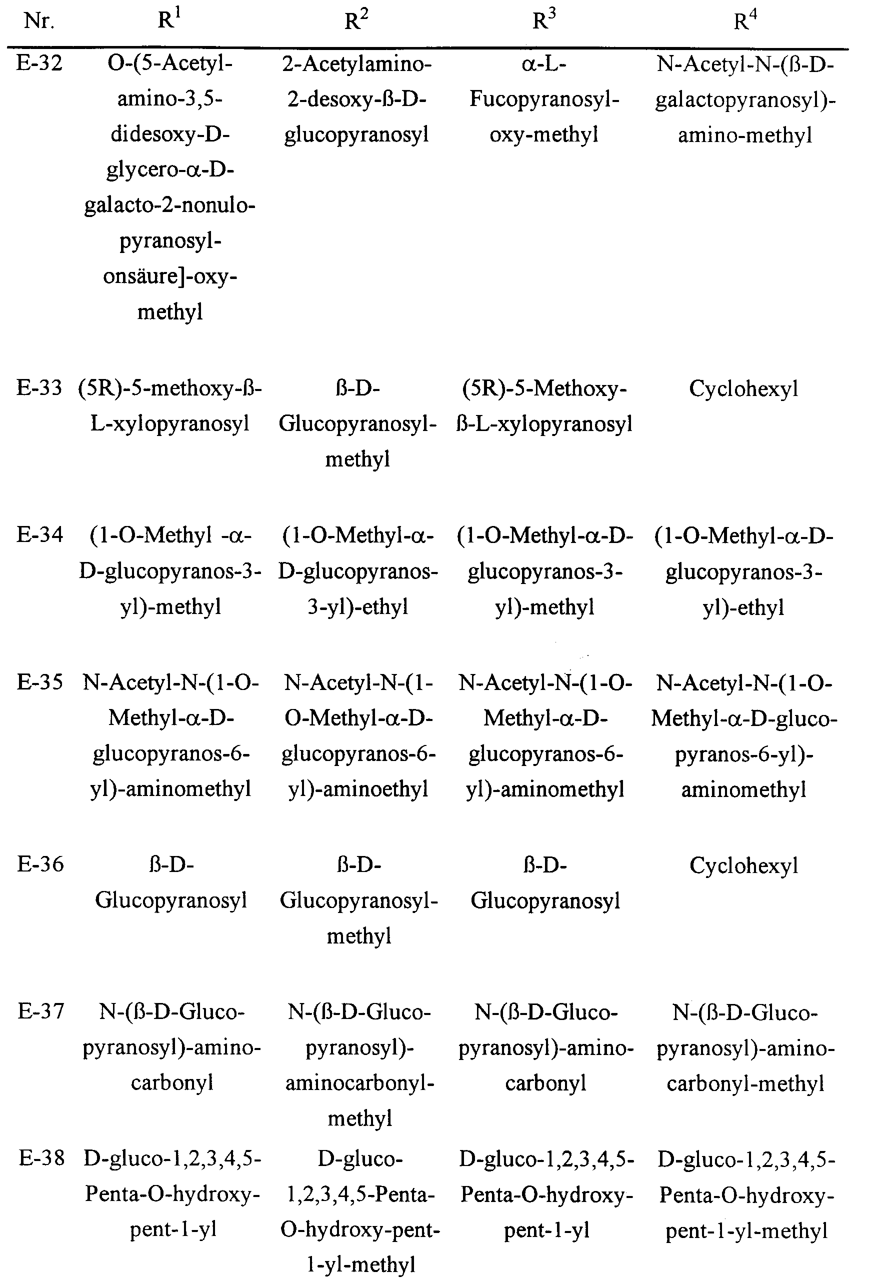

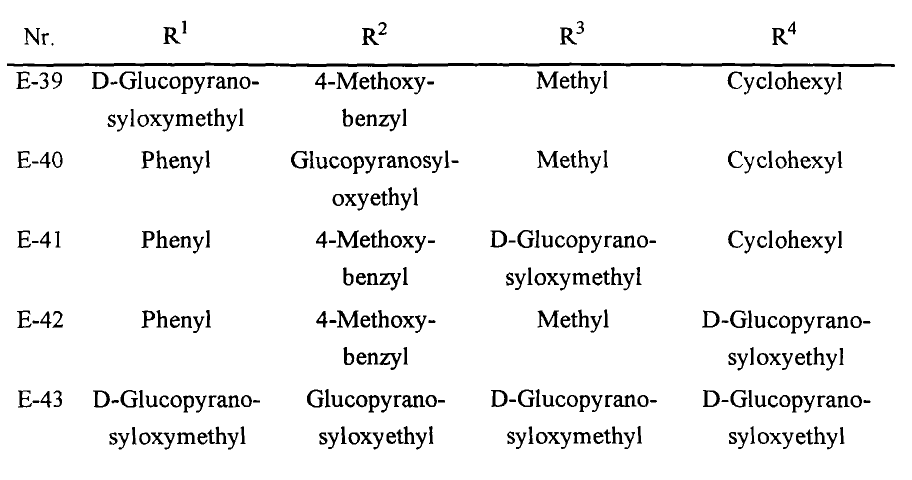

## Patentansprüche

1. Verbindungen der allgemeinen Formel (I) 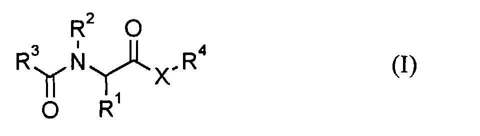 sowie pharmazeutisch akzeptable Salze davon,
in welcher
R¹ und R² gleich oder verschieden sein können und für einen gegebenenfalls substituierten, gesättigten oder einfach oder mehrfach ungesättigten Alkyl- oder Cycloalkylrest, einen gegebenenfalls substituierten Aryl oder Aralkylrest, einen gegebenenfalls substituierten, gesättigten oder einfach oder mehrfach ungesättigten oder aromatischen Heterocyclus oder Heterocyclyl-alkylrest oder einen Rest -Y-R⁵ stehen,
R³ und R⁴ gleich oder verschieden sein können und für Wasserstoff stehen oder die Bedeutung von R¹ aufweisen,
X für -O-, -NH-, -N(Niederalkyl)- oder -S- steht, wobei
Y für -(CH₂)ₙ-, -(CH₂)ₙ-O-, -(CH₂)ₙ-N(CO-R⁶)- oder -(CH₂)ₙ-CO-N(R⁷)- steht,
R⁵ für einen substituierten Alkyl-, Tetrahydrofuranyl-, Tetrahydropyranyl-, Niederalkyl-tetrahydrofuranyl- oder Niederalkyltetrahydropyranyl-ring steht und
R⁶ und R⁷ gleich oder verschieden sein können und für Wasserstoff oder Niederalkyl stehen und
n für eine Zahl von 0 bis 3 steht, mit der Maßgabe, daß (i) die Verbindungen der Formel (I) mindestens einen Rest -Y-R⁵ aufweisen müssen, und (ii) in dem Falle, in dem R² für einen Glycosylrest steht, in den Verbindungen der Formel (I) mindestens ein zusätzlicher Rest -Y-R⁵ vorhanden sein muß.

2. Verbindungen der Formel (I) gemäß Anspruch 1, in welcher
R⁵ für gegebenenfalls substituierte acyclische oder cyclische Kohlenhydratreste steht.

3. Verbindungen der Formel (I) gemäß Anspruch 1, in welcher R¹, R², R³ oder R⁴ für (i) Methyl, Ethyl, Propyl, iso-Propyl, Butyl, sek.-Butyl, tert.-Butyl, n-Hexyl, 5-Methyl-pentyl, 2-Ethyl-butyl, n-Octyl, 2-Ethyl-hexyl, n-Undecyl, n-Dodecyl, 1-Methyl-undecyl, 2,4-Dimethyl-decyl, 2,4,6-Trimethyl-nonyl oder n-Octadecyl; (ii) Vinyl, Allyl, But-2-enyl, But-3-enyl, Hex2-enyl, Hex-3-enyl, Hex-4-enyl, Hex-5-enyl, Oct-2-enyl, Oct-4-enyl, Oct-6-enyl, Dodec-2-enyl, Dodec-2,4-dienyl, Hexadec-8-enyl, Octadec-8-enyl oder Octadadec-2,4,6,8-tetraenyl; (iii) Cyclopropyl, Cyclobutyl, Cyclopentyl, Cyclohexyl, Cycloheptyl, Cyclooctyl, Bicylo[3.1.1]hept-3-yl, Octahydropentalen-2-yl oder Octahydropentalen-5-yl; (iv) Cyclopent-1-enyl, Cyclopent-2-enyl, Cyclohex-1-enyl, Cyclohex-2-enyl oder Cyclohex-3-enyl; (v) Phenyl, Naphthalenyl oder Anthracenyl; (vi) Benzyl, Naphthalen-1-yl-methyl, Naphthalen-2-yl-methyl, Naphthalen-1-yl-ethyl, Naphthalen-2-yl-ethyl, Naphthalen-1-yl-butyl oder Anthracen-1-yl-methyl; (vii) Pyrrolyl, Pyrrolinyl, Pyrrolidinyl, Furanyl, Tetrahydrofuranyl, Thiophenyl, Indolyl, Benzofuranyl, Benzothiophenyl, Carbazolyl, Pyrazolyl, Imidazolyl, Oxazolyl, Thiazolyl, Isoxazolyl, Isothiazolyl, Triazolyl, Tetrazolyl oder Thiadiazolyl; (viii) Pyridinyl, Dihydropyridinyl, Tetrahydropyridinyl, Pyranyl, Dihydropyranyl, Tetrahydropyranyl, Chinolinyl, Isochinolinyl, Acridinyl, Chromanyl, Pyridazinyl, Pyrimidinyl, Piperazinyl, Pyranzinyl, Phenazinyl, Phenoxazinyl, Phenothiazinyl, Morpholinyl, Thiazinyl, Phenothiazinyl, Triazinyl, Purinyl oder Pteridinyl; (ix) Pyrrolyl-methyl, Pyrrolyl-ethyl, Pyrrolyl-propyl, Furanyl-methyl, Imidazolyl-ethyl, Thiazolyl-ethyl, Pyridinyl-methyl, Chinolinyl-methyl oder Phenothiazinyl-ethyl steht. Jeder dieser Reste kann für sich geeignet substituiert sein, beispielsweise durch Niederalkyl, Aza- oder Oxa- oder Thia-niederalkylen, Halogen, Trichlormethyl, Trifluormethyl, Niederalkoxy, Amino, Mono- oder Di-niederalkyl-substituiertes Amino, Amino-niederalkyl, Mono- oder Di-niederalkyl-aminoalkyl, Formyl, Nieder-alkanoyl, Nieder-alkanoyloxy, Carboxy, Nieder-alkoxycarbonyl, Nitrilo, Aminocarbonyl, Mono- oder Di-niederalkyl-amino-carbonyl, Nitro, Hydroxy, Hydroxymethyl, Hydroxyethyl, Phenyl, substituiertes Phenyl, Mercapto, Alkylmercapto, Cyano, Pyridyl, Sulfonyl, Sulfonyloxy, Phosphoryl oder Phosphoryloxy. Zwei Ringglieder der Substituenten können auch überbrückt sein, beispielsweise durch Methylen, Ethylen, Alkylen, freies oder ketalisiertes Carbonyl, freies oder verethertes oder verestertes Hydroxymethylen, -S-, -O-, -NH-CO-NH-, -O-CO-NH-, -NHSO₂- oder -CONHSO₂-.

4. Verbindungen der allgemeinen Formel (I) gemäß Anspruch 1, in welcher R⁵ für acyclische oder cyclische Kohlenhydrate oder Kohlenhydratderivate steht, in denen eine oder mehrere Hydroxygruppen durch Schutzgruppen substituiert sein können oder gegen Aminogruppen, Acylaminogruppen, Halogenatome, Wasserstoffatome oder Mercaptogruppen ausgetauscht sein können oder zu Ketogruppen oder Carboxylatgruppen oxidiert sein können.

5. Verfahren zur Herstellung von Oligosaccharid-Analogen und Glykokonjugat-Analogen, dadurch gekennzeichnet, daß man Carbonylkomponenten, Carbonsäuren, Amine und Isonitrile, die zum Teil oder alle glycosyliert sind oder Kohlenhydratreste tragen, miteinander in einem Lösungsmittel reagieren läßt und das Produkt gewinnt.
